(19) Europäisches Patentamt
European Patent Office
Office européen des brevets

(11) EP 1 837 033 A1

(12) **EUROPEAN PATENT APPLICATION**
published in accordance with Art. 158(3) EPC

(43) Date of publication:
**26.09.2007 Bulletin 2007/39**

(21) Application number: **05805914.8**

(22) Date of filing: **14.11.2005**

(51) Int Cl.:
***A61K 45/00*** *(2006.01)* ***A61K 31/52*** *(2006.01)*
***A61K 38/00*** *(2006.01)* ***A61P 3/10*** *(2006.01)*
***A61P 43/00*** *(2006.01)* ***C12Q 1/02*** *(2006.01)*
***G01N 33/15*** *(2006.01)* ***G01N 33/50*** *(2006.01)*
***C07D 473/16*** *(2006.01)*

(86) International application number:
**PCT/JP2005/020870**

(87) International publication number:
**WO 2006/051951 (18.05.2006 Gazette 2006/20)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.11.2004 JP 2004329165**

(71) Applicants:
- **Tomizawa, Kazuhito**
  **Okayama-shi,**
  **Okayama 700-0921 (JP)**
- **Wei, Fanyan**
  **Okayama-shi, Okayama 700-0933 (JP)**
- **Matsui, Hideki**
  **Okayama-shi,**
  **Okayama 701-0211 (JP)**

(72) Inventors:
- **Tomizawa, Kazuhito**
  **Okayama-shi,**
  **Okayama 700-0921 (JP)**
- **Wei, Fanyan**
  **Okayama-shi, Okayama 700-0933 (JP)**
- **Matsui, Hideki**
  **Okayama-shi,**
  **Okayama 701-0211 (JP)**

(74) Representative: **Vossius & Partner**
**Siebertstrasse 4**
**81675 München (DE)**

(54) **REMEDY FOR DIABETES CONTAINING Cdk5 INHIBITOR**

(57) A therapeutic agent for diabetes containing a voltage-dependent L-type $Ca^{2+}$ channel regulator comprising, as an active ingredient, a cyclin-dependent kinase (hereafter abbreviated as Cdk) 5 inhibitor, an insulin secretion promoter comprising the regulator, a therapeutic agent for diabetes comprising the same are provided.

Fig. 12

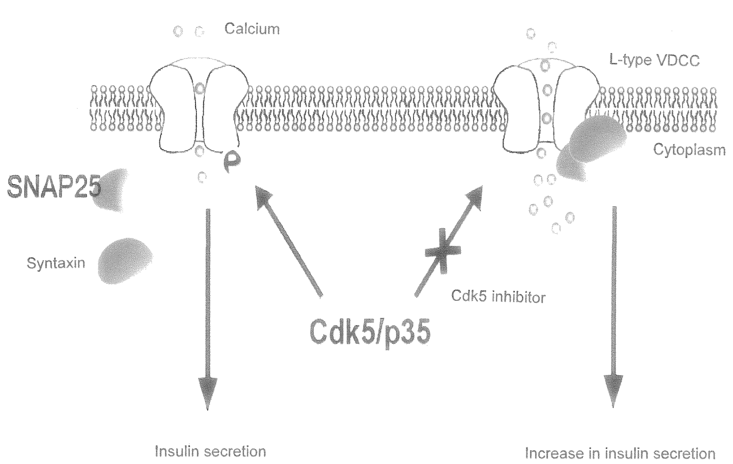


EP 1 837 033 A1

SEQUENCE LISTING

<110> Kazuhito Tomizawa; Fan-Yan Wei; Hideki Matsui

<120> A drug for treating diabetes comprising Cdk5 inhibitor

<130> PH-2647-PCT

<140>
<141>

<150> JP 2004/329165
<151> 2004-11-12
<160> 4

<170> PatentIn Ver. 2.1

<210> 1
<211> 307
<212> PRT
<213> Homo sapiens

<400> 1
Met Gly Thr Val Leu Ser Leu Ser Pro Ser Tyr Arg Lys Ala Thr Leu
1 5 10 15

Phe Glu Asp Gly Ala Ala Thr Val Gly His Tyr Thr Ala Val Gln Asn
20 25 30

Ser Lys Asn Ala Lys Asp Lys Asn Leu Lys Arg His Ser Ile Ile Ser
35 40 45

Val Leu Pro Trp Lys Arg Ile Val Ala Val Ser Ala Lys Lys Lys Asn
50 55 60

Ser Lys Lys Val Gln Pro Asn Ser Ser Tyr Gln Asn Asn Ile Thr His
65 70 75 80

Leu Asn Asn Glu Asn Leu Lys Lys Ser Leu Ser Cys Ala Asn Leu Ser
85 90 95

Thr Phe Ala Gln Pro Pro Pro Ala Gln Pro Pro Ala Pro Pro Ala Ser
100 105 110

Gln Leu Ser Gly Ser Gln Thr Gly Gly Ser Ser Ser Val Lys Lys Ala
115 120 125

Pro His Pro Ala Val Thr Ser Ala Gly Thr Pro Lys Arg Val Ile Val
130 135 140

Gln Ala Ser Thr Ser Glu Leu Leu Arg Cys Leu Gly Glu Phe Leu Cys
145 150 155 160

Arg Arg Cys Tyr Arg Leu Lys His Leu Ser Pro Thr Asp Pro Val Leu
165 170 175

Trp Leu Arg Ser Val Asp Arg Ser Leu Leu Leu Gln Gly Trp Gln Asp
180 185 190

Gln Gly Phe Ile Thr Pro Ala Asn Val Val Phe Leu Tyr Met Leu Cys
195 200 205

Arg Asp Val Ile Ser Ser Glu Val Gly Ser Asp His Glu Leu Gln Ala
210 215 220

Val Leu Leu Thr Cys Leu Tyr Leu Ser Tyr Ser Tyr Met Gly Asn Glu
225 230 235 240

Ile Ser Tyr Pro Leu Lys Pro Phe Leu Val Glu Ser Cys Lys Glu Ala
245 250 255

Phe Trp Asp Arg Cys Leu Ser Val Ile Asn Leu Met Ser Ser Lys Met
260 265 270

Leu Gln Ile Asn Ala Asp Pro His Tyr Phe Thr Gln Val Phe Ser Asp
275 280 285

Leu Lys Asn Glu Ser Gly Gln Glu Asp Lys Lys Arg Leu Leu Leu Gly
290 295 300

Leu Asp Arg
305

<210> 2

<211> 367
<212> PRT
<213> Homo sapiens

<400> 2
Met Gly Thr Val Leu Ser Leu Ser Pro Ala Ser Ser Ala Lys Gly Arg
1 5 10 15

Arg Pro Gly Gly Leu Pro Glu Glu Lys Lys Lys Ala Pro Pro Ala Gly
20 25 30

Asp Glu Ala Leu Gly Gly Tyr Gly Ala Pro Pro Val Gly Lys Gly Gly
35 40 45

Lys Gly Glu Ser Arg Leu Lys Arg Pro Ser Val Leu Ile Ser Ala Leu
50 55 60

Thr Trp Lys Arg Leu Val Ala Ala Ser Ala Lys Lys Lys Lys Gly Ser
65 70 75 80

Lys Lys Val Thr Pro Lys Pro Ala Ser Thr Gly Pro Asp Pro Leu Val
85 90 95

Gln Gln Arg Asn Arg Glu Asn Leu Leu Arg Lys Gly Arg Asp Pro Pro
100 105 110

Asp Gly Gly Gly Thr Ala Lys Pro Leu Ala Val Pro Val Pro Thr Val
115 120 125

Pro Ala Ala Ala Ala Thr Cys Glu Pro Pro Ser Gly Gly Ser Ala Ala

130 135 140

Ala Gln Pro Pro Gly Ser Gly Gly Gly Lys Pro Pro Pro Pro Pro Pro
145 150 155 160

Pro Ala Pro Gln Val Ala Pro Pro Val Pro Gly Gly Ser Pro Arg Arg
165 170 175

Val Ile Val Gln Ala Ser Thr Gly Glu Leu Leu Arg Cys Leu Gly Asp
180 185 190

Phe Val Cys Arg Arg Cys Tyr Arg Leu Lys Glu Leu Ser Pro Gly Glu
195 200 205

Leu Val Gly Trp Phe Arg Gly Val Asp Arg Ser Leu Leu Leu Gln Gly
    210                 215                 220

Trp Gln Asp Gln Ala Phe Ile Thr Pro Ala Asn Leu Val Phe Val Tyr
225                 230                 235                 240

Leu Leu Cys Arg Glu Ser Leu Arg Gly Asp Glu Leu Ala Ser Ala Ala
                245                 250                 255

Glu Leu Gln Ala Ala Phe Leu Thr Cys Leu Tyr Leu Ala Tyr Ser Tyr
            260                 265                 270

Met Gly Asn Glu Ile Ser Tyr Pro Leu Lys Pro Phe Leu Val Glu Pro
        275                 280                 285

Asp Lys Glu Arg Phe Trp Gln Arg Cys Leu Arg Leu Ile Gln Arg Leu
    290                 295                 300

Ser Pro Gln Met Leu Arg Leu Asn Ala Asp Pro His Phe Phe Thr Gln
305                 310                 315                 320

Val Phe Gln Asp Leu Lys Asn Glu Gly Glu Ala Ala Ala Ser Gly Gly
                325                 330                 335

Gly Pro Pro Ser Gly Gly Ala Pro Ala Ala Ser Ser Ala Ala Arg Asp
            340                 345                 350

Ser Cys Ala Ala Gly Thr Lys His Trp Thr Met Asn Leu Asp Arg
        355                 360                 365

<210> 3
<211> 23
<212> DNA
<213> Artificial Sequence

<220>
<223> Description of Artificial Sequence:Synthetic primer

<400> 3
gcccttcctg gtagagagct gta 23

<210> 4
<211> 23
<212> DNA
<213> Artificial Sequence

<220>
<223> Description of Artificial Sequence:Synthetic primer

<400> 4
ctgaccgctc tcattcttca agt 23

## Description

Technical Field

The present invention relates to a therapeutic agent for diabetes that causes few adverse effects such as hypoglycemic disorders or the like. Specifically, the present invention relates to a therapeutic agent for diabetes containing a voltage-dependent L-type $Ca^{2+}$ channel regulator comprising, as an active ingredient, a cyclin-dependent kinase (hereinafter abbreviated as Cdk) 5 inhibitor.

Background Art

Diabetes is induced by an absolute or relative deficiency of pancreatic insulin. It has been known that the most efficient diabetes therapies involve subcutaneous administration of human insulin. In particular, in cases of diabetic coma, insulin-dependent diabetes, and the like, a therapy involving subcutaneous administration of human insulin is absolutely necessary.

In addition, in order to normalize blood glucose levels and eliminate the presence of glucose in urine, oral hypoglycemic are used for moderate adult diabetes in cases in which the disease cannot be controlled by diet therapy and/or exercise therapy alone. As representative examples of oral hypoglycemic agents, sulfonylureas such as tolbutamide have been known Non-Patent Document 1 or the like).

Sulfonylureas restrain K channels suppressed by ATP in β cells of islets of Langerhans so as to depolarize β cells, resulting in the opening of voltage-dependent calcium channels. Thus, increases in intracellular calcium levels promotion of endogenous insulin secretion, resulting in decreases in blood sugar levels (see Non-Patent Document 1 or the like).

Since insulin and sulfonylureas have such action mechanisms, they are effective for diabetes, which is a metabolic disorder accompanied by symptoms such as hyperglycemia, glycosuria, excessive urination, excessive beverage intake, vascular disorders, and the like. In the cases of recent therapies as described above whereby hyperglycemia is ameliorated, ATP-sensitive $K^+$ ($K^+$ ATP) channels of pancreatic β cells are mainly targeted for increasing insulin secretion. However, according to such recent approaches, adverse effects involving hypoglycemia are often induced. Hypoglycemia induced by excessive insulin causes adverse effects such as disturbance of consciousness, convulsion, and palpitation, which have been problematic (see Non-Patent Document 2 or the like). These adverse effects cause brain damage. Thus, it is necessary to pay particular attention to such adverse effects.

Meanwhile, olomoucine is a compound having a structure represented by formula (III):

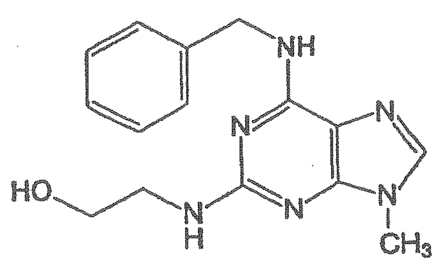

(III)

In general, olomoucine has been known as a cyclin-dependent inhibitor (see Non-Patent Document 3 or the like).

Recently, olomoucine has been used only in studies to inhibit synchronization in cell growth and DNA synthesis in lymphocytes stimulated by interleukin (see Non-Patent Document 3 or the like) with the use of the aforementioned action. There have been no reports of the use of olomoucine as a medicament.

Examples of a cyclin-dependent kinase that is inhibited by olomoucine include Cdk2/cyclin A ($IC_{50}$ = 7 μM), Cdk2/cyclin E ($IC_{50}$ = 7 μM), and Cdk5 ($IC_{50}$ = 3 μM). Among them, Cdk2/cyclin A and Cdk2/cyclin E are known to act exclusively in cells that normally proliferate and have no activity in differentiated pancreatic β cells (see Non-Patent Document 3 or the like). Meanwhile, Cdk5 exists in abundance mainly in differentiated nerve cells and pancreatic β cells. Functions of Cdk5 in nerve cells have been widely studied. Cdk5 is a serine/threonine kinase and is unevenly expressed in mammalian tissue (Tsai, LH. et al. Development 119, 1029-1040 (1993)). However, dominant expression of activators such as p35 and p39 in neurons results in limitation of kinase activity in neurons (Lew, J. et al, Nature 371, 423-426 (1994); Tsai, LH. et al. Nature 371, 419-423 (1994); and Tang, D. et al. J. Biol. Chem. 270, 26897-26903 (1995)). Cdk5 has multiple functions in neurons. Examples of such functions include neuronal migration, cortex formation, synaptic transmission, neuronal alteration, and a function related to habitual drug use (Cruz, JC, et al. Curr. Opin. Neurobiol. 14,

390-394 (2004); Bibb, JA. et al. Neurosignals 12, 191-199 (2003)). Very recently, researches have shown that p35 and p39 are expressed in pancreatic β cells (Ubeda, M. et al. Endocrinology 145, 3023-3031. (2004); and Lilja, L. et al. J. Biol. Chem. 279, 29534-29541 (2004)). In addition, Cdk5 exists in the cell membrane and cytoplasm of a β cell. Such localization is regulated based on glucose concentration. Specifically, it has been reported that Cdk5 binds to the cell membrane at low glucose concentrations; on the other hand, localization of Cdk5 is changed such that it is found in cytoplasm at high glucose concentrations (see Non-Patent Document 4). However, details of the action mechanism thereof have not been elucidated.

Non-Patent Document 1: "Stimulation of Insulin Release by RePaglinide and Glibenclamide Involves Both Common and Distinct Processes," Diabetes, 1998, 5, vol. 47, pp. 345 to 351
Non-Patent Document 2: "New drug targets for type 2 diabetes and the metabolic syndrome," Nature, 2001, 12, vol. 414, pp. 821 to 827
Non-Patent Document 3: "Inhibition of cyclin-dependent kinases by purine an alogues," European journal of biochemistry, 1994, vol. 224, pp. 771 to 786
Non-Patent Document 4: "Cyclin-dependent Kinase 5 Promotes Insulin Exocytosis," The journal of biological chemistry, 2001, vol. 276, 36, pp. 34199 to 34205

Disclosure of the Invention

In order to search for target molecules for diabetes treatment, the biochemical mechanism of insulin secretion has been widely examined. Insulin is secreted from pancreatic β cells in response to increases in blood glucose concentrations. Increases in intracellular ATP due to glucose metabolism causes closure of ATP-sensitive $K^+$ ($K^+$ ATP) channels, followed by cell membrane depolarization and opening of L-type voltage-dependent $Ca^{2+}$ channels (L-VDCCs) (Inagaki, N. et al. Science 270, 1166-1170 (1995); and Bratanova-Tochkova, TK. et al. Diabetes 51, S83-90 (2002)). Increases in $Ca^{2+}$ levels in cells induce SNARE (N-ethylmaleimide-sensitive attachment factor receptor) protein interaction and the subsequent exocytosis of granules comprising insulin (Gerber, SH. et al. Diabetes 51, S3-11 (2002)). $K^+$ ATP channel blockers such as sulfonylurea directly depolarize cell membranes. Thus, such blockers have been widely used to decrease high blood sugar levels. However, in cases of patients treated with such blockers, serious adverse effects based on a hypoglycemic mechanism have been observed (Moller, DE. Nature 414, 821-827 (2001)). Thus, a new approach depending on a physiological reaction has been long awaited.

It is an objective of the present invention to provide a hypoglycemic therapeutic agent that causes few adverse effects such as hypoglycemia disorders induced by existing insulin or insulin secretion promoters and is effective for diabetes, by solving the aforementioned problems so as to regulate voltage-dependent L-type $Ca^{2+}$ channels.

As a result of intensive studies to solve the aforementioned problems, the inventors of the present invention have found that Cdk5 inhibitors such as olomoucine and a peptidergic Cdk5 inhibitor can regulate voltage-dependent L-type $Ca^{2+}$ channels involved in insulin secretion. They also have found that such inhibitors are significantly useful as remedies for diabetes, such inhibitors causing no adverse effects involving hypoglycemia, promoting insulin secretion, and lowering blood glucose level to a normal value.

Specifically, they have found such inhibitors high-glucose-stimulated insulin secretion as a result of inhibition of cyclin-dependent kinase 5 (Cdk5)/p35 in MIN6 cells derived from rat pancreatic islets of Langerhans and pancreatic β cells of a p3 5-deficient mouse but do not increase insulin secretion at low glucose levels.

Kinase inhibition did not influence $Ca^{2+}$ influx via L-type voltage-dependent $Ca^{2+}$ channels (L-VDCCs) without glucose stimulation. Meanwhile, kinase inhibition enhanced inward whole-cell $Ca^{2+}$ currents upon high glucose stimulation in β cells, resulting in increases in $Ca^{2+}$ influx via VDCC. A molecular mechanism of promotion of Cdk5-dependent insulin secretion is a mechanism in which L-VDCC activity is increased by inhibiting Cdk 5. This is because Cdk5 phosphorylates $loop_{II\cdot III}$ (Ser783) of L-VDCC, and such phosphorylation decreases bonds between the loops and SNARE proteins (e.g., SNAP25 and syntaxin), resulting in decreases in L-VDCC activity. Based on these results, the present inventors have found that Cdk5/p35 can be used as a drug target that causes no adverse effects involving hypoglycemia, for the purpose of controlling hyperglycemia. This has led to the completion of the present invention.

That is, the present invention relates to a voltage-dependent L-type $Ca^{2+}$ channel regulator comprising a cyclin-dependent kinase (Cdk) 5 inhibitor, an insulin secretion promoter containing such regulator, and a therapeutic agent for diabetes containing the same.

Preferably, a Cdk5 inhibitor that is used in the aforementioned voltage-dependent L-type $Ca^{2+}$ channel regulator, the insulin secretion promoter comprising such regulator, and the therapeutic agent for comprising the same is olomoucine, roscovitine, purvalanol A, or a Cdk5 inhibitor containing any thereof, as an ATP-antagonistic Cdk5 inhibitor, a purine derivative Cdk5 inhibitor, or a peptidergic Cdk5 inhibitor.

Specifically, embodiments of the present invention are described as follows.

[1] A voltage-dependent L-type $Ca^{2+}$ channel regulator comprising, as an active ingredient, a cyclin-dependent kinase (Cdk) 5 inhibitor.

[2] The voltage-dependent L-type $Ca^{2+}$ channel regulator according to [1], such regulator not activating voltage-dependent L-type $Ca^{2+}$ channels upon hypoglycemia and promoting intracellular $Ca^{2+}$ influx via voltage-dependent L-type $Ca^{2+}$ channels upon hyperglycemia in cells of pancreatic islets of Langerhans.

[3] The voltage-dependent L-type $Ca^{2+}$ channel regulator according to [1] or [2], wherein the Cdk5 inhibitor is an ATP-antagonistic Cdk inhibitor.

[4] The voltage-dependent L-type $Ca^{2+}$ channel regulator according to [3], wherein the Cdk5 inhibitor is a purine derivative Cdk5 inhibitor.

[5] The voltage-dependent L-type $Ca^{2+}$ channel regulator according to [4], wherein the Cdk5 inhibitor is a compound represented by the following formula (I):

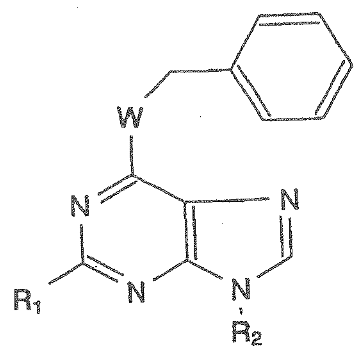

(I)

[wherein $R_1$ is $NY_1Y_2$, $Y_1$ and $Y_2$ independently represent H, $C_1$-$C_4$ alkyl, or $C_1$-$C_4$ hydroxyalkyl, $R_2$ represents H or $C_1$-$C_4$ alkyl, and W represents NH or O].

[6] The voltage-dependent L-type $Ca^{2+}$ channel regulator according to [4], wherein the Cdk5 inhibitor is a compound represented by the following formula (II):

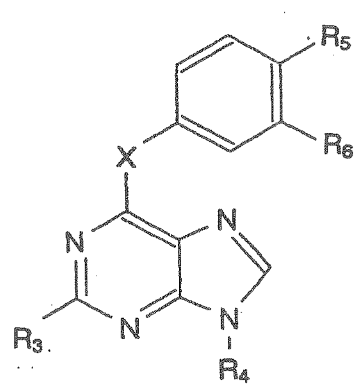

(II)

[wherein $R_3$ is $NZ_1Z_2$, $Z_1$ and $Z_2$ independently represent H, $C_1$-$C_4$ alkyl, or $C_1$-$C_4$ hydroxyalkyl, $R_4$ represents H or $C_1$-$C_4$ alkyl, $R_5$ represents H or $C_1$-$C_4$ alkyl, $R_6$ represents halogen, and X represents NH or O] .

[7] The $Ca^{2+}$ channel regulator according to [3] or [4], wherein the Cdk5 inhibitor is selected from the group consisting of olomoucine, roscovitine, purvalanol A, analogs thereof.

[8] The voltage-dependent L-type $Ca^{2+}$ channel regulator according to [1] or [2], wherein the Cdk5 inhibitor is a peptidergic Cdk5 inhibitor.

[9] The voltage-dependent L-type $Ca^{2+}$ channel regulator according to [8], wherein the peptidergic Cdk5 inhibitor is a fragment peptide of p35 or p39, which is a cyclin-dependent kinase activator.

[10] The voltage-dependent L-type $Ca^{2+}$ channel regulator according to [9], wherein the peptidergic Cdk5 inhibitor is a peptide having an amino acid sequence corresponding to the sequence from Cys at position 154 to Pro at position 279 of the amino acid sequence represented by SEQ ID NO: 1 or a peptide having an amino acid sequence derived from the amino acid sequence represented by SEQ ID NO: 1 by deletion, substitution, or addition of one or several amino acids.

[11] An insulin secretion promoter, which in cells of islets of Langerhans, such promoter comprising, as an active ingredient, the voltage-dependent L-type $Ca^{2+}$ channel regulator according to any one of [1] to [10].

[12] A therapeutic agent for diabetes comprising, as an active ingredient, the voltage-dependent L-type $Ca^{2+}$ channel regulator according to any one of [1] to [10].

[13] The therapeutic agent for diabetes according to [12], which does not cause hypoglycemic disorders to a subject to which the therapeutic agent has been administered.

[14] A method for screening for a therapeutic agent for diabetes *in vitro* based on intracellular $Ca^{2+}$ influx or increases in cellular insulin secretion induced by allowing a test substance that is a candidate Cdk5 inhibitor to act on cells of islets of Langerhans.

[15] The method according to [14], wherein the cells of islets of Langerhans are culture cells derived from pancreatic β cells.

[16] A method for screening for a therapeutic agent for diabetes *in vivo* based on increases in insulin secretion in pancreatic β cells upon hyperglycemia or decreases in blood glucose levels of an animal induced by administering a test substance that is a candidate Cdk5 inhibitor to a non-human animal.

Effects of the Invention

The voltage-dependent L-type $Ca^{2+}$ channel regulator, the insulin secretion inhibitor, and the therapeutic for diabetes according to the present invention each comprise a Cdk5 inhibitor as an active ingredient. Each thereof has a mechanism for regulating voltage-dependent L-type $Ca^{2+}$ channels so that they promote insulin secretion upon hyperglycemia but not upon hypoglycemia. Thus, they cause few adverse effects are effective in almost all diabetes cases unless all β cells are damaged. Thus, hyperglycemia can be ameliorated.

This description includes part or all of the contents as disclosed in the description and/or drawings of Japanese Patent Application No. 2004-329165, which is a priority document of the present application.

Brief Description of the Drawings

Fig. 1 shows effects of olomoucine upon insulin secretion levels obtained by stimulating isolated islets of Langerhans at low glucose concentrations.

Fig. 2 shows effects of olomoucine upon insulin secretion levels obtained by stimulating isolated islets of Langerhans at high glucose concentrations. In fig. 2, the symbol "*" represents $P < 0.05$.

Fig. 3 shows effects of roscovitine and olomoucine upon insulin secretion levels obtained by stimulating MIN6 cells at high glucose concentrations.

Fig. 4 shows effects of olomoucine and iso-olomoucine upon insulin secretion levels.

Fig. 5 shows a comparison between effects of olomoucine and those of glibenclamide at low glucose concentrations.

Fig. 6a shows comparison between Cdk5 activity in rat tissue and that in MIN6 cells.

Fig. 6b shows p35 mRNA relative expression levels of rat pancreatic islets of Langerhans and MIN6 cells.

Fig. 6c shows effects of olomoucine upon insulin secretion in MIN6 cells.

Fig. 6d shows effects of iso-olomoucine upon insulin secretion.

Fig. 6e shows effects of olomoucine upon glibenclamide-induced insulin secretion in KRB at a low glucose concentration (2.8 mM).

Fig. 6f shows effects of olomoucine upon glibenclamide-induced insulin secretion in KRB at a high glucose concentration (17.6 mM).

Fig. 6g shows inhibition of olomoucine-induced insulin secretion by diazoxide.

Fig. 6h shows of olomoucine upon forskolin-stimulated insulin secretion.

Fig. 7a-1 shows effects of olomoucine upon $Ca^{2+}$ kinetics in MIN6 cells stimulated with glucose on the basis of $Ca^{2+}$ concentration changes.

Fig. 7a-2 shows effects of olomoucine upon $Ca^{2+}$ kinetics in MIN6 cells stimulated with glucose on the basis of maximum $Ca^{2+}$ influxes.

Fig. 7b-1 shows effects of olomoucine upon $Ca^{2+}$ kinetics in MIN6 cells stimulated with glibenclamide on the basis of $Ca^{2+}$ concentration changes.

Fig. 7b-2 shows effects of olomoucine upon $Ca^{2+}$ kinetics in MIN6 cells stimulated with glibenclamide on the basis of maximum $Ca^{2+}$ influxes.

Fig. 7c shows the current-voltage relationship of whole-cell $Ca^{2+}$ currents via VDCCs of MIN6 cells.

Fig. 7d shows whole-cell $Ca^{2+}$ currents of a single β cell isolated from a wild type mouse (black circles) and those of a single β cell isolated from a p35 KO mouse (white circles).

Fig. 8a shows preserved $loop_{II \cdot III}$ domains, which are underlined (single or double underline). In fig. 8a, double-underlined portions represent motifs that are considered to be phosphorylated by Cdk5.

Fig. 8b shows phosphorylation of a GST conjugate of $Ca_v1.2$ (754a.a.-900a.a.)-derived $loop_{II \cdot III}$ or $loop_{II \cdot III}$ comprising alanine mutated from serine783.

Fig. 8c shows results of Western blotting for measurement of $loop_{II \cdot III}$ phosphorylation with the use of a specific antibody against phosphorylated serine783.

Fig. 8d shows results of immunoprecipitation of lysates of MIN6 cells treated with and not treated with olomoucine.

Fig. 8e shows results of Western blotting for detection of proteins bound to phosphorylated GST-$loop_{II \cdot III}$ obtained after incubation of such loop with synataxin 1A or SNAP-25.

Fig. 8f shows effects of phosphorylation of $loop_{II \cdot III}$ domains upon interaction with syntaxin and SNAP-25.

Fig. 9a shows glucose-stimulated insulin secretion in isolated islets of Langerhans.

Fig. 9b shows plasma insulin levels of wild type mice (black circles) and $p35^{-/-}$ mice (white circles).

Fig. 9c shows plasma glucose changes in response to intraperitoneal glucose challenge in wild type mice (black circles) and p35 KO mice (white circles).

Fig. 10 shows the structure of voltage-dependent L-type $Ca^{2+}$ channel.

Fig. 11 shows an insulin secretion mechanism.

Fig. 12 shows a mechanism for controlling insulin secretion upon regulation of VDCC-SNARE interaction by Cdk5.

Fig. 13 shows changes in blood glucose levels in diabetes mouse models after administration of roscovitine.

Fig. 14 shows changes in body weights of diabetes mouse models after administration of roscovitine.

## Best Mode for Carrying Out the Invention

The present invention is hereafter described in detail.

Examples of the Cdk5 inhibitor used in the invention include an ATP-antagonistic Cdk5 inhibitor and a purine derivative Cdk5 inhibitor. Preferably, it is possible to use a peptidergic Cdk5 inhibitor. An ATP-antagonistic inhibitor is a compound that inhibits Cdk5 activity by antagonizing ATP. A purine derivative is a derivative obtained via substitution of various portions of purine and purine nucleus. Examples of such ATP-antagonistic inhibitor and such purine derivative Cdk5 inhibitor include olomoucine, roscovitine, and purvalanol A described below.

For instance, as a purine derivative Cdk5 inhibitor, it is possible to use a compound represented by the following formula (I) or (II).

In formula (I), $R_1$ preferably represents $NH_2$ or $NCH_2CH_2OH$, $R_2$ preferably represents $CH_2$ or $CH(CH_3)C_2H_5$, and W preferably represents NH:

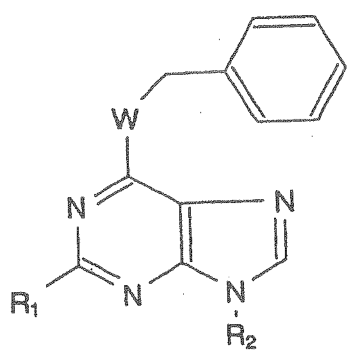

(I)

[wherein $R_1$ is $NY_1Y_2$, $Y_1$ and $Y_2$ independently represent H, $C_1$-$C_4$ alkyl, or $C_1$-$C_4$ hydroxyalkyl, $R_2$ represents H or $C_1$-$C_4$ alkyl, and W represents NH or O] .

In formula (II), $R_3$ preferably represents $NH_2$ or $NCH_2CH_2CH(CH_3)OH$, $R_4$ preferably represents $CH_2$, $R_5$ preferably represents H or $CH(CH_3)_2$, $R_6$ preferably represents Cl, and X preferably represents NH:

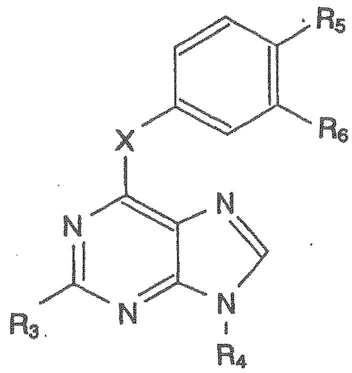

(I I)

[wherein $R_3$ is $NZ_1Z_2$, $Z_1$ and $Z_2$ independently represent H, $C_1$-$C_4$ alkyl, or $C_1$-$C_4$ hydroxyalkyl, $R_4$ represents H or $C_1$-$C_4$ alkyl, $R_5$ represents H or $C_1$-$C_4$ alkyl, $R_6$ represents halogen, and X represents NH or O].

Specific examples of a compound represented by formula (I) include olomoucine, roscovitine, and analogs thereof. Specific examples of a compound represented by formula (II) include purvalanol A, purvalanol B, compound 52, and analogs thereof. Chemical formulae of olomoucine, roscovitine, purvalanol A are shown as (III), (IV) and (V), respectively, as follows.

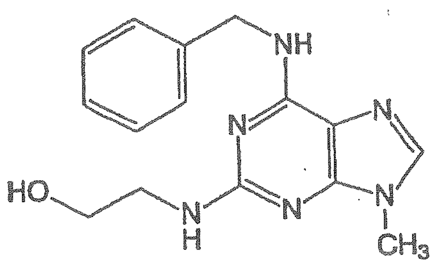

(I I I)

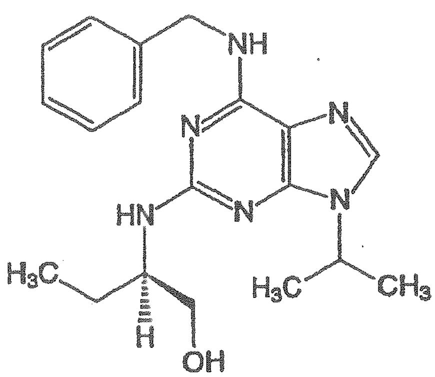

(I V)

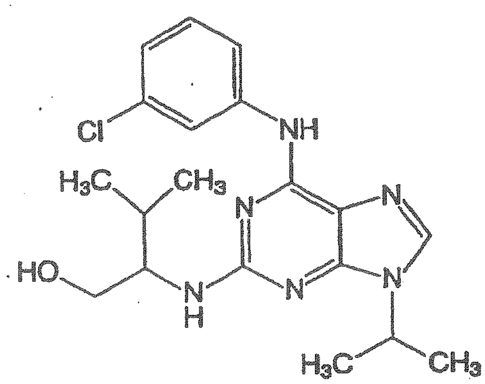

(V)

Herein, the term "analog" indicates a compound having a structure analogous to that of a compound having Cdk5 inhibitory action, inhibiting Cdk5 enzyme activity, and having an action similar to that of such compound. The expression "structure analogous to" is used for a structure in which the structure of a portion exhibiting Cdk5 inhibitory action remains unchanged and a group that does not influence such structure is substituted with another group having a size that is not significantly different from the size of such group, for example.

The present invention encompasses prodrugs of the aforementioned compounds having Cdk5 inhibitory action. The term "prodrug" is referred to as a compound capable of being converted into the Cdk inhibitor of the present invention via hydrolysis or the like of such prodrug in a living body to which such prodrug has been administered. In addition, the Cdk5 inhibitor of the present invention encompasses pharmaceutically acceptable salts of the above compounds.

Further, another example of the Cdk5 inhibitor that can be used in the present invention is a peptidergic Cdk5 inhibitor. Such peptidergic Cdk5 inhibitor is a peptide that binds to Cdk5. Examples thereof include peptide of cyclin-dependent kinase activator proteins. These peptidergic Cdk5 inhibitors can be obtained by for peptides that bind to Cdk5 by, for example, a yeast two-hybrid method. Examples of the Cdk5 inhibitor of the present invention include a p35 (CDK5R1)-derived peptide fragment (e.g., Ya-Li Zheng et al. Eur. J. Biochem. 26, 4427-4434 (2002)) and a p39 (CDK5R2)-derived peptide fragment. The full-length amino acid sequences of p35 and p39 are represented by SEQ ID NOS: 1 and 2, respectively. A peptide fragment that binds to Cdk5 of a peptide comprising such full-length amino acid sequence but does not activate Cdk5 can be used as a Cdk5 inhibitor. A minimal peptide having p35 activity is a peptide (p16) having an amino acid sequence corresponding to the sequence from Pro at position 138 to Asn at position 291 of the amino acid sequence represented by SEQ ID NO: 1. Examples of the Cdk5 inhibitor include a peptide derived from such peptide (p16) by deletion or substitution of one amino acid to several tens of amino acids in the amino acid sequence. An example thereof is a peptide having an amino acid sequence corresponding to the sequence from Glu at position 150 to Ser at position 287 of the amino acid sequence represented by SEQ ID NO: 1, such peptide being derived from the peptide (p16) having an amino acid corresponding to the sequence from Pro at position 138 to Asn at position 291 of the amino acid sequence by deletion of 11 amino acids of the N terminus and 4 amino acids of the C terminus. Further, it is also possible to preferably use a peptide having an amino acid sequence corresponding to the sequence from Cys at position 154 to Pro at position 279 of the amino acid sequence represented by SEQ ID NO: 1, for example.

Furthermore, it is also possible to use, as a peptidergic Cdk5 inhibitor, a peptide having an amino acid sequence derived from the amino acid sequence of a peptide having Cdk5 inhibitory action by deletion, substitution, or addition of one or several amino acids. Herein, the term "one or several amino acids" means not more than 10 amino acids, preferably not more than 5 amino acids, further preferably not more than 3 amino acids, and particularly preferably not more than 2 amino acids. Such peptidergic Cdk inhibitor has high biocompatibility and is unlikely to cause adverse effects.

It is possible to determine whether or not a candidate compound or a candidate peptide has Cdk5 inhibitory action and to determine $IC_{50}$ of a candidate compound or a candidate peptide relative to that of Cdk5 by the following method.

It is possible to determine $IC_{50}$ of Cdk5 in a manner such that $\gamma^{32}$p-ATP, which is a radioactive isotope, is added as a tracer to a compound or a candidate peptide; histone H1, which serves as a substrate, is added to a solution containing Cdk5 and its activation subunit (p35 or p25 (limited proteolysis product of p35)); and incorporation of $\gamma^{32}$p by histone H1 in the presence of a Cdk5 inhibitor at a different concentration is measured.

According to the present invention, a Cdk5 inhibitor acts on Cd25 that exists in β cells so as to inhibit Cdk5 enzyme activity. When glucose is incorporated into cells of pancreatic islets of Langerhans, ATP is synthesized, increased ATPs act on the cell membrane, and $K^+$ channels are closed, followed by depolarization. Thereafter, voltage-dependent

L-type $Ca^{2+}$ channels are opened influx of $Ca^{2+}$ from the extracellular side takes place, resulting in insulin secretion. At such time, when voltage-dependent L-type $Ca2^{+}$ channels are opened as a result of cell membrane depolarization, SNARE proteins (e.g., SNAP25 and syntaxin) bind to $loops_{II\cdot III}$of voltage-dependent L-type $Ca^{2+}$ channels so that $Ca^{2+}$ influx can readily take place, resulting in insulin secretion. Such $loop_{II\cdot III}$is referred to as a loop structure portion that is formed between domains II and III of a voltage-dependent L-type $Ca^{2+}$ channel in a cell (fig. 10). Cdk5 phosphorylates serine at position 783 of the amino acid sequence of $loop_{II\cdot III}$ and inhibits binding of SNARE proteins to the loops, Accordingly, $Ca^{2+}$ influx is inhibited, resulting in suppression of insulin secretion (fig. 11). The Cdk5 inhibitor of the present invention inhibits phosphorylation of the above serine, regulates voltage-dependent L-type $Ca^{2+}$ channels, and promotes $Ca^{2+}$ influx via voltage-dependent L-type $Ca^{2+}$ channels such that insulin secretion is promoted (fig. 12). As described above, β cell depolarization is induced by glucose stimulation. Upon hypoglycemia, cell membrane depolarization does not take place, and thus voltage-dependent L-type $Ca^{2+}$ channels are not opened. Thus, there are no influences, regardless of the presence or absence of Cdk5 inhibition. Meanwhile, upon hyperglycemia, depolarization takes place and voltage-dependent L-type $Ca^{2+}$ channels are opened. At such time, insulin secretion is promoted by inhibiting Cdk5 so as to inhibit phosphorylation as described above. In the above manner, the inhibitor used in the present invention regulates voltage-dependent L-type $Ca^{2+}$ channels exclusively upon hyperglycemia so as to promote insulin secretion. Thus, the Cdk5 inhibitor used in the present invention promotes insulin secretion exclusively upon hyperglycemia but not upon hypoglycemia. Therefore, such inhibitor does not cause hypoglycemia disorders. Herein, the term "hypoglycemia" means a condition in which blood sugar levels are below normal or roughly normal values. In such condition, the blood glucose concentration is not more than 100 mg/dL, preferably not more than 90 mg/dL, further preferably 80 mg/dL, and particularly preferably 70 mg/dL.

Diabetes is classified into two types: insulin-dependent diabetes and non-insulin-dependent diabetes. Insulin-dependent diabetes appears in juveniles due to absolute deficiency of insulin in lesions of β cells of pancreatic islets of Langerhans. Non-insulin-dependent diabetes is induced by deterioration of insulin responsiveness of somatic cells and/or decreases in insulin secretion due to obesity and/or mutation. A Cdk5 inhibitor promote insulin secretion by acting on β cells. Thus, unless all β cells are damaged, it is expected that significant therapeutic effects can be obtained in all diabetes cases.

Examples of administration routes of the therapeutic agent of the present invention include percutaneous, intravenous, intramuscular, subcutaneous, and oral administration routes. Oral administration before eating is preferable because the therapeutic agent can immediately act upon hyperglycemia. In addition, local administration to the pancreas may be performed. For local administration to the pancreas, the drug of the present invention may be injected directly into the pancreas or the vicinity thereof. For instance, the drug may be injected directly into pancreatic tissue by modification involving the application of a hollow needle to an endoscope used for pancreas treatment. Further, a percutaneous needle can be used for transabdominal injection into pancreatic tissue.

Dosage forms of the aforementioned therapeutic agent can adequately be determined based on administration routes. Specific examples of dosage forms include tablets and liquids such as aqueous solutions, emulsions, and suspensions. In general, oral administration is preferable, and thus aqueous solutions or tablets are preferable.

Dosages can adequately be determined depending upon the route of administration, upon the patient's age and weight, and upon the severity of the malady. When a Cdk5 inhibitor is a purine derivative inhibitor, the dosage in terms of active ingredient for single administration is preferably 0.0003 to 0.03 mg/kg body weight and more preferably 0.003 to 0.01 5 mg/kg body weight. A exceeding such level may also be administered. For instance, the dosage for administration may be 1 to 1000 mg/kg body weight, 20 to 500 mg/kg body weight, or 50 to 500 mg/kg body weight. When the dosage is less than 0.0003 mg/kg body weight, it might be impossible to obtain effects of a therapeutic agent for diabetes. In addition, when a Cdk5 inhibitor is a peptidergic inhibitor, the dosage in terms of an active ingredient for a single administration is preferably 0.005 to 5 mg/kg body weight and more preferably 0.005 to 1 mg/kg body weight.

Upon formulation of the therapeutic agent of the invention, various types of additives that are usually used by persons skilled in the art can be used depending on dosage form. Examples of such additives include diluents, isotonizing agents, carriers, and pH stabilizers.

In the specific example of an administration method involving intravenous infusion, the dosage of the aforementioned therapeutic agent in terms of active ingredient for a single administration may be 0.003 to 0.015 mg/kg body weight. Such dosage may be increased for administration. For instance, the dosage for administration may be 1 to 1000 mg/kg body weight, 20 to 500 mg/kg body weight, or 50 to 500 mg/kg body weight. In terms of the dosage of a peptidergic Cdk5 inhibitor, the inhibitor is dissolved in a physiological saline solution or the like so as to be administered at 0.005 to 5 mg/kg body weight, corresponding to the administration of 10 to 50 μM of the resulting solution. Upon intramuscular injection, the dosage of an active ingredient for a single administration may be 0.003 to 0.015 mg/kg body weight. Thus, in terms of the dosage of a peptidergic Cdk5 inhibitor, the inhibitor is dissolved in a physiological saline solution or the like so as to be administered at 0.005 to 5 mg/kg body weight. Preferably, administration is carried out before eating three times per day.

Hypoglycemic effects of the Cdk5 inhibitor of the present invention can be evaluated by the amount of insulin

secreted upon glucose administration to culture cells derived from isolated islets of Langerhans and those derived from pancreatic β cells. In addition, effects also can be evaluated by measuring insulin secretion levels or decreases in blood upon administration of the inhibitor to non-human animals. With the use of such measurement methods, it is possible to screen for a Cdk5 inhibitor that can be used as an active ingredient of the voltage-dependent L-type $Ca^{2+}$ channel regulator of the present invention. That is, when a test substance that is a candidate Cdk inhibitor is allowed to act on culture cells derived from pancreatic β cells such as islets of Langerhans that have been isolated *in vitro,* isolated pancreatic cells, and rat MIN6 cells having capacity of secreting insulin, it is possible to determine whether or not the test substance can be used as an active ingredient of the voltage-dependent L-type $Ca^{2+}$ channel regulator of the present invention based on promotion of intracellular $Ca^{2+}$ influx or promotion of cellular insulin secretion due to regulation of cellular voltage-dependent L-type $Ca^{2+}$ channels. In such case, there is no limitation in terms of an animal from which islets of Langerhans, pancreatic β cells, and culture cells derived from pancreatic β cells are derived. Examples of such cells include cells of islets of Langerhans derived from humans, mice, rats, and the like. In addition, when a test substance is administered to a non-human animal preferably a diabetes mouse model having a high blood glucose level, it is possible to determine whether or not the test substance can be used as an active ingredient of the voltage-dependent L-type $Ca^{2+}$ channel regulator of the present invention based on increases in levels of insulin secretion from pancreatic β cells of the animal or decreases in blood glucose levels of the animal. In particular, such determination may be carried out based on the condition that a test substance the amount of insulin secreted upon hyperglycemina so as to decrease blood glucose level, while on the other hand, it does not increase the amount of insulin secreted upon hypoglycemia so as not to decrease blood glucose level. In such case, examples of non-human animals used include, but are not limited to, mice and rats.

Examples

The present invention is hereafter described in greater detail with reference to the following examples, although the technical scope of the present invention is not limited thereto.

Example 1

Action of olomoucine at low glucose concentrations

(1) Preparation of islets of Langerhans

In accordance with Goto et al. (Transplantaion, vol. 43, pp. 725 to 730, 1987), islets of Langerhans were isolated from 6-week-old male Wister rats. The rats were etherized so as to be subjected to thoracotomy. The common bile duct of each rat was removed and a collagenase solution (320 U/ml, Sigma) was slowly injected into the pancreas. Then, the swollen pancreas was excised. The collagenase-containing pancreas was digested at 37˚C in a water bath for 30 minutes, followed by dispersion with the use of a pipet. Thereafter, islets of Langerhans were isolated based on the concentration gradient of a Ficoll solution (Amersham Pharmacia). The islets of Langerhans were incubated while being kept at 37˚C for 30 minutes in a Ringer solution (119 mM sodium chloride, 4.74 mM potassium chloride, 1.19 mM sodium dihydrogen phosphate, 25 mM sodium bicarbonate, 10 mM HEPES, 2.54 mM calcium chloride, 1.19 mM magnesium chloride, and 0.2% BSA) saturated with a mixed gas of 95% $O_2$ and 5% $CO_2$.

(2) Action of olomoucine

The above islets of Langerhans (15 islets per set) were selected under a stereoscopic microscope (Olympus Corporation) and separately added to Ringer solutions (3.3 mM glucose, 0.5 ml each) containing olomoucine (Sigma) at a different concentration as shown in fig. 1, followed by incubation for 30 minutes. Each Ringer solution was replaced with a stimulation solution, which was a Ringer solution (0.5 ml) having the same glucose and olomoucine concentrations. 30 minutes after replacement, insulin contained in each stimulation solution was detected using an insulin detection kit (Ab Bead Insulin, Eiken Chemical Co., Ltd.) in accordance with protocols of the kit. The experiment was conducted for each concentration group (n = 6).

As shown in fig. 1, insulin concentrations under the condition of 3.3 mM glucose were almost equivalent, regardless of the presence of olomoucine. That is, at low glucose concentrations, olomoucine had no influence at all upon insulin secretion of islets of Langerhans. This result indicates that application of olomoucine upon hypoglycemia never causes further hypoglycemia leading to severe adverse effects.

Example 2

Action of olomoucine at high glucose concentrations

Islets of Langerhans (15 islets per set), which had been prepared in a manner similar to that used in Example 1 (1), were selected under a stereoscopic microscope (Olympus Corporation) and separately added to Ringer solutions (3.3 mM glucose, 0.5 ml each) each containing olomoucine (Sigma) at a different concentration as shown in fig. 2, followed by incubation for 30 minutes. Each Ringer solution was replaced with a stimulation solution, which was 0.5 ml of a Ringer solution with a high glucose concentration (17.3 mM glucose) and with the same olomoucine concentration. 30 minutes after replacement, insulin contained in each stimulation solution was detected using an insulin detection kit (Ab Bead Insulin, Eiken Chemical Co., Ltd.) in accordance with protocols of the kit. The experiment was conducted for each concentration group (n = 6).

As shown in fig. 2, the insulin secretion levels in the presence of olomoucine significantly increased compared with the level in the absence of olomoucine. In addition, the insulin secretion levels increased depending on increases in olomoucine concentrations. These results indicate that it is possible to cope with diabetes cases with different symptoms by controlling olomoucine concentrations.

Example 3

Insulin secretion promotion action of roscovitine

(1) Culture of insulin-secreting cells (MIN6)

MIN6 cells capable of secreting insulin (provided by Professor Susumu Seino, Doctral Program in Advanced Biomedical Applications, Department of Cellular and Molecular Medicine, Graduate School of Medicine, Chiba University, 1-33, Yayoi-cho, Inage-ku, Chiba-shi, Chiba, Japan) were cultured in accordance with the method of Mae et al. (American Journal of Physiological Endocrinal Metabolism, 2000, vol. 274, pp. 773 to 781). MIN6 cells ($5 \times 10^5$) were in a DMEM medium (GIBCO) and on a 35-mm culture dish (Corning Incorporated), followed by culture for 2 days in an incubator (5% $CO_2$, SANYO).

(2) Action of roscovitine

First, culture solutions of the MIN6 cells cultured in (1) above were separately replaced with 0.5 ml of a Ringer solution with a low glucose concentration (3.3 mM glucose) containing 20 μM roscovitine (Calbiochem) and with 0.5 ml of a Ringer solution with a low glucose concentration (3.3 mM glucose) containing 20 μM olomoucine (Sigma), followed by incubation for 30 minutes. Subsequently, the resulting solutions were replaced with the following stimulation solutions, respectively: 0.5 ml of a Ringer solution having a high glucose concentration (17.6 mM glucose) and containing roscovitine at the concentration; and 0.5 ml of a Ringer solution having a high glucose concentration (17.6 mM glucose) and containing olomoucine at the same concentration (n = 6 for each group). A Ringer solution containing neither roscovitine nor olomoucine was used as a control (n = 6). After culture for 2 hours, insulin contained in medium was detected using an insulin detection kit (Ab Insulin, Eiken Chemical Co., Ltd.) in accordance with protocols for the kit.

As shown in fig. 3, insulin secretion of MIN6 cells was promoted in the presence of roscovitine. This result indicates that roscovitine also has an action of promoting insulin secretion as with the case of olomoucine, both roscovitine and olomoucine serving as Cdk5 inhibitors.

Experimental example 1

Islets of Langerhans (15 islets per set), which had prepared in a manner similar to that used in Example 1 (1), were selected under a stereoscopic microscope (Olympus Corporation) and separately added to a Ringer solution (3.3 mM glucose, 0.5 ml each) containing 10 μM olomoucine and to such a Ringer solution containing 10 μM iso-olomoucine (Calbiochem), followed by incubation for 30 minutes. Subsequently, the resulting solutions were replaced with the following stimulation solutions, respectively: 0.5 ml of a Ringer solution having a low glucose concentration (3.3 mM glucose) and containing olomoucine (Sigma) at the same concentration; and 0.5 ml of a Ringer solution having a low glucose concentration (3.3 mM glucose) and containing iso-olomoucine (Galbiochem) at the same concentration (n = 6 for group). A Ringer solution containing neither olomoucine nor iso-olomoucine was used as a control (n=6). 30 minutes after replacement, insulin contained in each stimulation solution was using an insulin detection kit (Ab Bead Insulin, Eiken Chemical Co., Ltd.) in accordance with protocols for the kit.

Iso-olomoucine is an isomer of olomoucine but does not have a Cdk5 inhibitory effect even though it has a

structure analogous to that of olomoucine. With the use of iso-olomoucine, it was confirmed that the action of olomoucine influences insulin secretion promotion effects. Fig. 4 shows the results. The structure of iso-olomoucine is as shown in the following formula (VI).

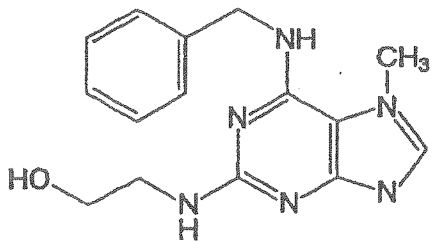

(V I)

In the presence of olomoucine (10 μM), the Ringer solution with a high glucose concentration significantly promoted insulin secretion. However, iso-olomoucine (10 μM, which is an isomer of olomoucine, did not promote insulin secretion at all. This result indicates that the Cdk5 inhibitory effect of olomoucine promotes insulin secretion.

Experimental example 2

Islets of Langerhans (15 islets per set), which had been prepared in a manner similar to that used in Example 1 (1), were selected under a stereoscopic microscope (Olympus Corporation). Then, controls (n = 6), samples A (n = 6), samples B (n = 6), and samples C (n = 6) were prepared. Controls and samples A were added to Ringer solutions (3.3 mM glucose, 0.5 ml each) and samples B and samples C were added to Ringer solutions (0.5 ml each) containing 10 μM olomoucine, followed by incubation for 30 minutes. The Ringer solution used for controls was replaced with the following stimulator: 0.5 ml of a Ringer solution having a low glucose concentration (3.3 mM glucose) containing neither olomoucine nor glibenclamide. The ringer solutions used for samples A, B, and C were replaced with the following stimulators, respectively: 0.5 ml of a Ringer solution having a low glucose concentration (3.3 mM glucose) and containing 200 nM glibenclamide; 0.5 ml of a Ringer solution having a low glucose concentration (3.3 mM glucose) and containing 10 μM olomoucine; and 0.5 ml of a Ringer solution having a low glucose concentration (3.3 mM glucose) and containing 200 nM glibenclamide and 10 μM olomoucine, 30 minutes after replacement, insulin contained in each stimulation solution was detected using an insulin detection kit (Ab Bead Insulin, Eiken Chemical Co., Ltd.) in accordance with protocols for the kit. Further, islets of Langerhans were subjected to ultrasonication for cell disruption, and insulin contents of islets of Langerhans were detected using an insulin detection kit (Ab Bead Insulin, Eiken Chemical Co., Ltd.) in accordance with protocols for the kit in a manner similar to that used above.

Regarding effects obtained at the low glucose concentration, olomoucine was compared with glibenclamide, which is a sulfonylurea used as a therapeutic agent for diabetes based on the insulin secretion percentage relative to the content of insulin in islets of Langerhans. In addition, the insulin secretion percentage was calculated by the following formula.

$$\text{Insulin secretion percentage} = \frac{\text{Amount of insulin secreted } (\mu\text{U/ml})}{\text{Amount of insulin in islets of Langerhans } (\mu\text{U/ml})} \times 100\ (\%)$$

Fig. 5 shows the results.

Glibenclamide caused a significant increase in insulin secretion in islets of Langerhans at the low glucose concentration. However, olomoucine did not influence insulin secretion. The results indicate that, unlike a therapeutic agent such as a sulfonylurea, olomoucine does not cause hypoglycemia at all and promotes insulin secretion exclusively at a high glucose concentration.

Example 4

Molecular mechanism of insulin secretion promotion using a cyclin-dependent kinase 5 inhibitor

This Example was carried out in the described below.

Cell culture and Ca $^{2+}$ imaging

MIN6 cells were cultured in a DMEM containing 15% fetal bovine serum, 25 mM glucose, 71 mM 2-mercaptoethanol, and 2 mM glutamine (Lilla, V. et al. Endocrinology 144, 1368-1379 (2003); and Minami, K. et al. Am. J. Physiol. Endocrinol. Metab. 279, 773-781 (2000)). The cells ($1 \times 10^6$) were seeded on a 35-mm glass bottom dish (IWAKI, Tokyo, Japan), followed by cell proliferation for 48 hours. For $Ca^{2+}$ imaging, the cells were preincubated at 37˚C for 2 hours in a modified Krebs-Ringer bicarbonate HEPES buffer solution (KRB: 125 mM Nacl, 4.74 mM KCl, 1 mM $CaCl_2$, 1.2 M $KH_2PO_4$, 5 mM $NaHCO_3$, 25 mM HEPES (pH 7.4), and 0.1% BSA) containing 2.8 mM glucose. In the presence of 50 μM olomoucine (Sigma), 5 μM fura-2/acetoxymethyl (Molecular Probe) was added to the cells for 30 minutes, followed by washing twice. The resulting solution was replaced with KRB containing 17.6 mM glucose or KRB containing 100 nM glibenclamide. The $Ca^{2+}$ level of the cells was using an Axiovert 200 inverted microscope (Carl Zeiss MicroImaging) by a dual excitation wavelength method (340/380).

Electrophysiological analysis

Pancreatic islets of Langerhans were isolated by a collagenase digestion method (Wollheim, CB. et al. Methods Enzymol. 192, 188-223. (1990)). Dispersed cells of islets of Langerhans were cultured in DMEM supplemented with 10% fetal bovine serum on Cellocate coverslips (Eppendorf) in a 35-mm dish. The cells were incubated at 37˚C for 24 to 72 hours prior to an experiment. The recording of a single whole pancreatic β cell was carried out by a conventional method (Beguin, P. et al. Nature 411, 701-706 (2001)). After the experiment, the pancreatic β cells were identified by immunostaining with the use of anti-insulin antibodies.

Measurement of insulin secretion

MIN6 cells ($0.4 \times 10^6$) were seeded on a 24-well dish (IWAKI) before use and allowed to stand for 48 hours. Next, the cells were incubated for 2 hours with the use of KRB containing 2.8 mM glucose and further incubated for 30 minutes with the use of KRB containing olomoucine or KRB not containing olomoucine. Then, the cells were stimulated for 1 hour with the use of various types of secretion promoters in the presence or absence of olomoucine. The insulin concentrations were measured by radioimmunoassay (Eiken Chemical Co., Ltd.) according to the manufacturer's protocols.

Production of p35-deficient mice

P35 knockout mice were produced and maintained (background: 129/Sv × C57BL/6J) (Ohshima, T. et al. Proc. Natl. Acad. Sci. U. S. A. 98, 2764-2769 (2001); Ohshima, T. et al. Genomics 35, 372-375 (1996)).

Intraperitoneal glucose tolerance test

After fasting overnight, glucose (2g/Kg) was intraperitoneally administered to the mice and blood was collected from each of them 15, 30, 60, and 120 minutes after administration. The glucose concentrations were measured using a glucometer (NIPRO). The insulin levels were measured by enzyme immunoassay (Pharmacia Biosciences).

$Loop_{II-III}$ phosphorylation

Recombinant cDNA encoding $Loop_{II \cdot III}$ of a human α1C subunit of VDCC was subcloned into pGEX6P (Amersham Bioscience). Serine783 was mutated into alanine according to the manufacturer's instructions (Stratagene). Purified recombinant proteins were phosphorylated *in vitro* with the use of Cdk5/p35 (Upstate) by a conventional method (Tomizawa, K. et al. J. Cell. Biol. 163, 813-824 (2003)). In order to measure intracellular phosphorylation of $loop_{II \cdot III}$ caused by Cdk5, MIN6 cells were treated with olomoucine for 30 minutes and homogenized with the use of a dissolution buffer (50 mM tris-HCl (pH 7.5), 5 mM $MgCl_2$, 1 mM EGTA, 1 mM EDTA, 1% CHAPS, 1 μM FK506, 100 nM okadaic acid, and a protease inhibitor cocktail). α1C subunits were immunoprecipitated from the cell lysate with the use of anti-α1C subunit antibodies (Sigma, C1603). The expression of the phosphorylated α1C subunit at Ser783 was measured

using phosphorylation-specific antibodies obtained from serum of a rabbit to which CLARTApSPEKK peptide had been injected. The total amount of the subunits was detected by Western blotting analysis with the use of anti-α1C subunit antibodies (Chemicon).

Relative gene expression

The quantitative analysis of p35mRNA was carried out using an ABI PRISM 7000 Sequence Detection System (Applied Biosystems) according to the manufacturer's instructions. In short, total RNA was isolated using an RNeasykit (Qiagen) from a rat brain, pancreatic islets of Langerhans, and MIN6 cells. The total RNA was subjected to synthesis of the first strand of cDNA with the use of an oligo dT primer (Invitrogen). For the relative quantification of p35 mRNA, measurement was carried out using an ABI Prism 7000 sequence detection system (Applied Biosystems) and p35-specific primers (sense: GCCCTTCCTGGTAGAGAGCTGTA (SEQ ID NO: 3); and antisense: CTGACCGCTCTCATTCTTCAAGT (SEQ ID NO: 4)). The copy number of FKBP mRNA was determined using TaqMan FKBP Control Reagents (Applied Biosystems) so that the same could be designated as an internal control. The cDNA copy number of p35 mRNA was normalized relative to the result for FKBP with a sample volume equivalent to that of p35 MRNA. Quantitative analysis was carried out using the analysis software (7000 v 1.1 (Applied Biosystems).

Statistical analysis

Data was shown on the basis of the mean value (± S. E. M.). The data was analyzed by Student' t-test or ANOVA used for comparison of two conditions. Then, the results were compared with those obtained under different conditions. The level of significance was $P < 0.05$.

Accordingly, the following facts were reveled.

In this Example, high levels of Cdk5 activity and p35 expression were observed in pancreatic β cells and MIN6 cells of a β-cell-derived cell line (figs. 6a and 6b).

In order to examine physiological functions of Cdh5 in pancreatic β cells, MIN6 cells were treated with olomoucine (membrane-permeable Cdk5 inhibitor) (Vesely, J. et al. Eur. J. Biochem, 224, 771-786 (1994)). The cells were preincubated for 30 minutes with the use of olomoucine at different concentrations. The cells were stimulated for 1 hour with 16.7 mM glucose. Then, insulin secretion into a medium was measured. It is interesting that olomoucine promoted high-glucose-stimulated insulin secretion in a dose-dependent manner. Meanwhile, under low glucose conditions, insulin secretion was not promoted by olomoucine-induced Cdk5 inhibition (fig. 6c). Further, iso-olomoucine (a structural isomer of olomoucine) did not influence insulin secretion (fig. 6d). Induction of insulin secretion due to Cdk5 inhibition was also confirmed when roscovitine (another conventional Cdk5 inhibitor) was used (data not shown).

Glibenclamide, which is a $K^+$ ATP channel blocker and has been widely used for diabetes treatment, directly induced membrane depolarization and then induced insulin secretion without high-glucose stimulation (fig. 6e: control: 42.5 ± 2.9 μg/h; and glibenclamide: 116.3 ± 1.0 μg/h). Olomoucine significantly promoted insulin secretion induced by glibenclamide (fig. 6e: glibenclamide + olomoucine: 278.8 ± 28.7 μg/h). Such additive action of olomoucine was observed in the cases of high glucose levels (fig. 6f: glibenclamide: 412.5 ± 35.7 μg/h; and glibenclamide + olomoucine: 636.3 ± 35.9 μg/h). Olomoucine-dependant promotion of high-glucose-stimulated insulin secretion was completely suppressed by diazoxide ($K^+$ATP channel opener) (fig. 6g). Insulin secretion is stimulated by two main pathways: a $K^+$ATP channel-dependent pathway and a $K^+$ATP channel-nondependent pathway in which cyclic AMP is an important mediator (Renstrom, E. et al. J. Physiol. 502, 105-118 (1997)). Next, it was examined whether or not Cdk5 would control cyclic AMP-dependent insulin secretion. Forskolin induced insulin secretion in MIN6 cells without high-glucose stimulation. Olomoucine did not influence insulin secretion induced by forskolin (fig. 6h: forskolin: 122.7 ± 9.3; and forskolin ± olomoucine: 136.5 ± 5.3 ($P > 0.05$)). These results indicate that Cdk5 controls glucose-stimulated insulin secretion downstream of $K^+$ATP channels. Cdk5 inhibition promotes high-glucose-stimulated insulin secretion but does not promote insulin secretion under low glucose conditions.

Figs. 6a to 6h show that Cdk5 inhibition promotes insulin secretion stimulated by glucose in MIN6 cells.

Fig. 6a shows comparison of Cdk5 activity in MIN6 cells and different types of rat tissue. Cdk5 was immunoprecipitated from each tissue (shown in fig. 6a) and from MIN6 cells with the use of anti-Cdk5 antibodies (SantaCruz, C8). The kinase activity was measured using histone H1 as a substrate.

Fig. 6b shows the relative expression of p35 mRNA in rat pancreatic islets of Langerhans and in MIN6 cells. The p35 mRNA expression levels were normalized relative to the level of FKBP, The obtained expression levels were compared with the expression level in a rat brain by relative quantitative PCR.

Fig. 6c shows effects of olomoucine upon insulin secretion in MIN6 cells.

Cells were treated with olomoucine at concentrations shown in fig. 6c for 30 minutes, followed by stimulation for 1 hour with the use of KRB containing 2.8 mM glucose (white bar) or 17.6 mM glucose (gray bar) in the presence of olomoucine. Insulin concentrations were measured by RIA (n = 6, *$P < 0.01$ (**$P < 0.001$) for each group).

Fig. 6d shows effects of iso-olomoucine upon insulin secretion.

MIN6 cells were separately treated with 20 $\mu$M olomoucine or 20 $\mu$M iso-olomoucine. Comparison of insulin secretion was carried out in the manner used for fig. 6c (n=5, *P < 0.01 for each group).

Figs. 6e and 6f show effects of olomoucine upon glibenclamide-induced insulin secretion in KRB at a low glucose concentration (2.8 mM (fig. 6e)) or at a high glucose concentration (17.6 mM (fig. 6f)) (n=5, *P < 0.01 (**P < 0.001) for each group).

Fig. 6g shows inhibition of olomoucine-induced insulin secretion caused by diazoxide.

Cells were treated in the presence or absence of 50 $\mu$M olomoucine for 30 minutes, followed by stimulation of insulin secretion induced by 17.6 mM glucose in the presence of 100 $\mu$M diazoxide (n = 6 for each group).

Fig. 6h shows effects of olomoucine upon forskolin-stimulated insulin secretion.

Cells were preincubated with 50 $\mu$M olomoucine for 30 minutes, followed by stimulation of insulin secretion induced by 10 $\mu$M forskolin in KRB containing 2.8 mM glucose (n = 6 for each group).

After the closure of $K^+$ATP channels in pancreatic $\beta$ cells, $Ca^{2+}$ influx via L-VDCC subsequently takes place (Bratanova-Tochkova, TK. et al. Diabetes 51, S83-90 (2002)). Cdk5 might influence $Ca^{2+}$ influx following glucose stimulation. $Ca^{2+}$ kinetics in MIN6 cells subjected to stimulation induced by glucose and glibenclamide were examined by $Ca^{2+}$ imaging. Olomoucine did not influence $Ca^{2+}$ influxes in the absence of glucose and glibenclamide (figs. 7a-1, 7a-2, 7b-1, and 7b-2). However, $Ca^{2'}$ influxes induced by glucose (114.4 $\pm$ 2.1%, figs. 7a-1 and 7a-2) and glibenclamide (129 $\pm$ 4.5%, figs. 7b-1 and 7b-2) were promoted in the presence of olomoucine. These results indicate that increases in insulin secretion due to Cdk5 inhibition resulted from $Ca^{2+}$ influxes enhanced by olomoucine. In order to examine whether or not olomoucine would have directly influenced L-VDCC activity, effects of a Cdk5 inhibitor upon whole-cell $Ca^{2+}$ currents were examined with the use of MIN6 cells. Cdk5 inhibition that had been induced by olomoucine enhanced inward $Ca^{2+}$ currents, which were induced during application of step pulses from -40 mV to +60 mV (fig. 7c). Peak $Ca^{2+}$ currents (-20.1 $\pm$ 1.59 A/F) induced by depolarization to 10 mV in olomoucine-treated MIN6 cells were obviously higher than those of control cells (-15.1 $\pm$ 1.67 A/F). Further, in order to confirm control of $Ca^{2+}$ currents by Cdk5, $Ca^{2+}$ currents of $\beta$ cells isolated from a p35-knockout mouse having a very low level of Cdk5 kinase activity were examined (fig. 7d). $Ca^{2+}$ currents induced by depolarization to 20 mV in $\beta$ cells of a p35-deficient mouse were enhanced compared with those of a wild type mouse (p35-deficient: -18.9 $\pm$ 2.16 A/F; and wild type: -27.4 $\pm$ 3.48 A/F). These results suggest that Cdk5 plays an important role in control of VDCCs in pancreatic $\beta$ cells.

Fig. 7a-1 to 7d show effects of olomoucine upon $Ca^{2+}$ mobilization in MIN6 cells.

During examination shown in figs. 7a-1, 7a-2, 7b-1, and 7b-2, 5 $\mu$M Fura-2 was added to the cells for 30 minutes in the presence or absence of 50 $\mu$M olomoucine. After washing, the cells were stimulated with 17.6 mM glucose (figs. 7a-1 and 7a-2) or 100 nM glibenclamide (figs. 7b-1 and 7b-2). $Ca^{2+}$ concentrations were presented as percentages of 340/380 nm fluorescent emission. The graphs (fgs. 7a-1 and 7b-1) show representative traces of changes in $Ca^{2+}$ concentration. The graphs (figs. 7a-2 and 7b-2) show the maximum $Ca^{2+}$ influxes. Data for each of 20 to 30 cells were collected after each experiment. The mean value of the total values was calculated and normalized every 3 to 4 experiments (*P < 0.01).

Fig. 7c shows the current-voltage relationship of whole-cell $Ca^{2+}$ currents via VDCCs of MIN6 cells in the presence (black circles) or absence (white circles) of 50 $\mu$M olomoucine (n = 15 (*P < 0.04) for each group).

Fig. 7d shows whole-cell $Ca^{2+}$ currents of a single $\beta$ cell isolated from a wild type mouse (black circles) and those of a single $\beta$ cell isolated from a p35 KO mouse (white circles) (n = 18 (*P < 0.05) for each group).

Next, an accurate mechanism, of Cdk5-dependent $Ca^{2+}$ influx control via VDCCs in pancreatic $\beta$ cells was examined. Changes in $Ca^{2+}$ channel currents have not been known. However, Cdk5 has been reported to phosphorylate $\alpha$1A subunits of P/Q-type $Ca^{2+}$ channels in neurons (Tomizawa, K. et al. J. Neurosci. 22, 2590-2597 (2002)). Protein kinase A (PKA) and protein kinase C (PKC) phosphorylate cardiac isoforms of and $NH_2$ termini of a VDCC $\alpha$1C subunit (De, Jongh, KS. et al. Biochemistry 35, 10392-10402 (1996); Gao, T. et al. Neuron 19, 185-196 (1997); and McHugh D et al. Proc. Natl. Acad. Sci. U. S. A. 97, 12334-12338 (2000)). In pancreatic $\beta$ cells, such $\alpha$1C subunits are calcium channels and they are thus necessary for insulin secretion (Schulla, V. et al. EMBO. J. 22, 3844-3854 (2003)). However, $NH_2$- and COOH-terminus domains of L-VDCC $\alpha$1C subunits in pancreatic cells do not have amino acid sequences are preferably selected by Cdk5. The present inventors discovered that intracellular $Loop_{II-III}(L_{II-III})$in such subunit is phosphorylated *in vitro* by Cdk5. A GST-fusion mouse $L_{II-III}$is a favorable substrate for Cdk5 *in vitro* (fig. 8b). An amino acid sequence (SPEK) at Ser783 of $L_{II-III}$ is a sequence that is preferably selected by Cdk5. Such has maintained in mammals and zebrafishes. Thus, Cdk5 was considered to phosphorylate Ser783 of $L_{II-III}$(fig. 8a). Cdk5-dependent phosphorylation was blocked in a mutant $L_{II-III}$in which Ser783 had been converted into Ala (fig. 8b). In order to examine whether or not Cdk5 would phosphorylate an $\alpha$1C subunit at Ser783 of L-VDCC in a pancreatic $\beta$ cell, a specific antibody against phosphorylated Ser783 was produced (fig. 8c). Then, phosphorylation in MIN6 cells was examined using such antibody. In the cells, a decrease in phosphorylation was observed in the presence of a Cdk5 inhibitor. On the other hand, Ser783 was phosphorylated in the absence of olomoucine (fig. 8d). N- and P/Q-type VDCCs associate with several proteins involved in the exocytosis mechanism, such proteins including 25 kDa (SNAP-25) and syntaxin (synaptosome proteins).

In neurons, several such proteins comprise a SNARE complex containing synaptobrevin, which is a vesicular protein (Yokoyama, CT. et al. J. Neurosci. 17, 6929-6938 (1997)). Interaction (synprint) sites of synaptic proteins are localized in intracellular loops II-III ($L_{II \cdot III}$) of VDCCs. Phosphorylation modulates the aforementioned association. In the present invention, it was examined whether or not phosphorylation of Ser783 would have influenced interaction involving SNARE proteins, such as SNAP-25 and syntaxin 1a *in vitro*. Purified syntaxin and SNAP-25 were incubated with GST- $L_{II \cdot III}$ in the presence or absence of Cdk5/p35. Then, the interaction was examined by Western blotting analysis (fig. 8e). In the presence of Cdk5/p35 and ATP, binding between $L_{II \cdot III}$ and syntaxin and that between $L_{II \cdot III}$ and SNAP-25 were inhibited (fig. 8e). Inhibitory effects of Cdk5-dependent phosphorylation of $L_{II \cdot III}$ upon SNARE protein interaction were further confirmed with the use of extracts of MIN6 cells. GST-$L_{II \cdot III}$, which had been phosphorylated by Cdk5/p35, was incubated with a cell lysate so that interaction involving SNAP-25 and syntaxin was examined. Phosphorylation of $L_{II \cdot III}$ by Cdk5 significantly inhibited binding involving SNAP-25 and syntaxin (fig. 8e). These results indicate that Cdk5 phosphorelates $L_{II \cdot III}$ of L-VDCC in pancreatic β cells so that interaction involving SNARE proteins is prevented.

Figs. 8a to 8f show effects of phosphorylation of the $loop_{II \cdot III}$ domain upon interaction involving syntaxin and SNAP-25.

In fig. 8a, preserved $loop_{II \cdot III}$ domains are underlined (single or double underlines). Double-underlined portions represent motifs that are considered to be phosphorylated by Cdk5.

For the examination shown in fig. 8b, a GST conjugate of $Ca_v1.2$ (754a.a.-900a.a.)-derived $loop_{II \cdot III}$ or $loop_{II \cdot III}$ comprising alanine mutated from serine783 were phosphorylated with Cdk5/p35 at 32°C during the time course shown in fig. 8b. Incorporation of $^{32}$P-ATP was measured by Cherenkov counting (c.p.m.).

For the examination shown in fig. 8c, wild-type $loop_{II \cdot II}$ or an alanine mutant (S783A) of $loop_{II \cdot III}$ was phosphorylated with Cdk5/p35 for 1 hour. Phosphorylation of $loop_{II \cdot II}$ was measured by Western blotting with the use of a specific antibody against phosphorylated serine783.

For the examination shown in fig. 8d, MIN6 cells treated with and not treated with 50 μM olomoucine were dissolved. Immunoprecipitation of $Ca_v1.2$ was carried out. Phosphorylation of serine 783 was measured by Western blotting.

For the examination shown in fig. 8e, GST-$loops_{II \cdot III}$ (100 pmol) immobilized on glutathione beads were phosphorylated with Cdk5/p35 and incubated with synataxin 1A (50 pmol) or SNAP-25 (50 pmol). Proteins bound to the loops were detected by Western blotting.

For the examination shown in fig. 8f, phosphorylated- and unphosphorylated-GST-$loops_{II \cdot III}$ were used for coprecipitation of SNAP-25 and syntaxin from an MIN6 dissolution solution.

Effects of a Cdk5 inhibitor upon insulin secretion from rat pancreatic islets of Langerhans were examined. The results obtained by using MIN6 cells (fig. 6a) indicate that olomoucine promoted glucose-stimulated insulin secretion in a dose-dependent manner in isolated islets of Langerhans; however, the inhibitor did not influence secretion at low glucose levels (fig. 9a). Lastly, it was examined whether or not Cdk5 inhibition would promote *in vivo* insulin secretion. An intraperitoneal glucose tolerance test was carried out with the use of p35-deficient mice. Blood insulin levels (0.56 ± 0.07 ng/ml) ofp35-deficient mice 15 minutes after glucose injection were higher than those (0.34 ± 0.06 ng/ml) of wild type mice (fig. 9b). Further, in the cases of p35-deficient mice, significant decreases in blood glucose levels glucose administration were observed compared with the cases of the wild type mice (fig. 9c).

Figs. 9a to 9c show that Cdk5 inhibition promotes insulin secretion in isolated islets of Langerhans and *in vivo*.

For the examination shown in fig. 9a, insulin secretion stimulated with glucose in isolated islets of Langerhans was examined in the presence of olomoucine at concentrations shown in fig. 9a (n = 6 for each group (*P < 0.05)).

Fig. 9b shows plasma insulin levels of wild type mice (black circles) and those of $p35^{-/-}$ mice (white circles) measured during the time course shown in fig. 9b (n = 6 for each group (*P < 0.05)).

For the examination shown in fig. 9c, changes in plasma glucose levels in response to intraperitoneal glucose challenge (2g/Kg body weight) were examined with the use of wild type mice (black circles) and p35 KO mice (white circles) (n = 6 for each group (*P < 0.05)).

Based on the above examination, a novel signal cascade controlled by glucose stimulation in pancreatic β cells was found. Cdk5 inhibition promoted insulin secretion *in vitro* and *in vivo* in response to increased glucose concentrations. Effects of such promotion were derived from changes in L-VDCCs via control of phosphorylation at synprint sites and interaction involving SNARE proteins. A molecular mechanism for controlling Cdk5-dependent insulin secretion was presented by the examination. Further, the results of the examination demonstrate that Cdk5 may be a new treatment target for diabetes.

Example 5

Hypoglycemic effects shown in diabetes mouse models KK-Ay

8-week-old ale KK-Ay mice (32 individuals, CLEA Japan, Inc.) were divided into groups (8 individuals each)

with the proviso that there would be no differences among groups in terms of blood glucose levels, Hypoglycemic effects of roscovitine (LC Laboratories), which is a CDK5 inhibitor, were examined.

In order to prepare roscovitine at doses of 1 mg/kg, 10 mg/kg, and 100 mg/kg, a suspension (0.5% methylcellulose/physiological saline solution) was used. Then, subcutaneous administration of roscovitine was carried out once daily for 2 consecutive weeks. A solvent alone was administered to a control group. Blood glucose levels were measured one day before day 0 and on days 3, 7, 10, and 14 with the use of a blood glucose monitor (Free Style, NIPRO).

Fig. 13 shows time-dependent changes in blood glucose levels of mice of each group. Fig. 14 shows time-dependent changes in body weights of mice of each group. In fig. 13, the unit of the vertical axis is indicated by "mg/dL." In fig. 14, the unit of the vertical axis is indicated by "g."

Accordingly, obvious hypoglycemic effects were not observed in groups to which roscovitine had been administered at 1 mg/kg or 10 mg/kg. However, statistically significant decreases in blood glucose levels were observed in a group to which roscovitine had administered at 100 mg/kg.

**Claims**

1. A voltage-dependent L-type $Ca^{2+}$ channel regulator comprising, as an active ingredient, a cyclin-dependent kinase (Cdk) 5 inhibitor.

2. The voltage-dependent L-type $Ca^{2+}$ channel regulator according to claim 1, such regulator not activating voltage-dependent L-type $Ca^{2'}$ channels upon hypoglycemia and promoting intracellular $Ca^{2+}$ influx via voltage-dependent L-type $Ca^{2+}$ channels upon hyperglycemia in cells of pancreatic islets of Langerhans.

3. The voltage-dependent L-type $Ca^{2+}$ channel regulator according to claim 1 or 2, wherein the Cdk5 inhibitor is an ATP-antagonistic Cdk inhibitor.

4. The voltage-dependent L-type $Ca^{2+}$ channel regulator according to claim 3, wherein the Cdk5 inhibitor is a purine derivative Cdk5 inhibitor.

5. The voltage-dependent L-type $Ca^{2+}$ channel regulator according to claim 4, wherein the Cdk5 inhibitor is a compound represented by the following formula (I):

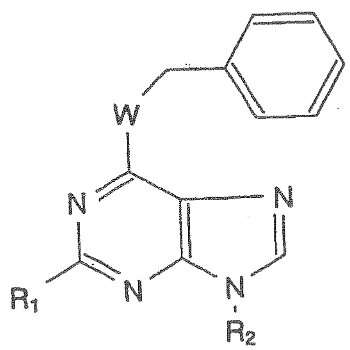

(I)

[wherein $R_1$ is $NY_1Y_2$, $Y_1$ and $Y_2$ independently represent H, $C_1$-$C_4$ alkyl, or $C_1$-$C_4$ hydroxyalkyl, $R_2$ represents H or $C_1$-$C_4$ alkyl, and W represents NH or O] .

6. The voltage-dependent L-type $Ca^{2+}$ channel regulator according to claim 4, wherein the Cdk5 inhibitor is a compound

represented by the following formula (II):

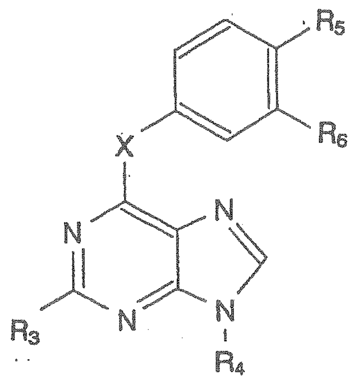

(I I)

[wherein $R_3$ is $NZ_1Z_2$, $Z_1$ and $Z_2$ independently represent H, $C_1$-$C_4$ alkyl, or $C_1$-$C_4$ hydroxyalkyl, $R_4$ represents H or $C_1$-$C_4$ alkyl, $R_5$ represents H or $C_1$-$C_4$ alkyl, $R_6$ represents halogen, and X represents NH or O] .

**7.** The $Ca^{2+}$ channel regulator according to claim 3 or 4, wherein the Cdk5 inhibitor is selected from the group consisting of olomoucine, roscovitine, purvalanol A, and analogs thereof.

**8.** The voltage-dependent L-type $Ca^{2+}$ channel regulator according to claim 1 or 2, wherein the Cdk5 inhibitor is a peptidergic Cdk5 inhibitor.

**9.** The voltage-dependent L-type $Ca^{2+}$ channel regulator according to claim 8, wherein the peptidergic Cdk5 inhibitor is a fragment peptide of p35 or p39, which is a cyclin-dependent kinase activator.

**10.** The voltage-dependent L-type $Ca^{2+}$ channel regulator according to claim 9, wherein the peptidergic Cdk5 inhibitor is a peptide having an amino acid sequence corresponding to the sequence from Cys at position 154 to Pro at position 279 of the amino acid sequence represented by SEQ ID NO: 1 or a peptide having an amino acid sequence derived from the amino acid sequence represented by SEQ ID NO: 1 by deletion, substitution, or addition of one or several amino acids.

**11.** An insulin secretion promoter, which acts in cells of islets of Langerhans, such promoter comprising, as an active ingredient, the voltage-dependent L-type $Ca^{2+}$ channel regulator according to any one of claims 1 to 10.

**12.** A therapeutic for diabetes comprising, as an active ingredient, the voltage-dependent L-type $Ca2^{+}$ channel regulator according to any of claims 1 to 10.

**13.** The therapeutic for diabetes according to claim 12, which not cause hypoglycemic disorders to a subject to which the therapeutic has been administered.

**14.** A method for screening for a therapeutic agent for diabetes *in vitro* based on intracellular $Ca^{2+}$ influx or increases in cellular insulin secretion induced by allowing a test substance that is a candidate Cdk5 inhibitor to act on cells of islets of Langerhans.

**15.** The method according to claim 14, wherein the cells of islets of Langerhans are culture cells derived from pancreatic β cells.

**16.** A method for screening for a therapeutic agent for diabetes *in vivo* based on increases in insulin secretion in pancreatic β cells upon hyperglycemia or decreases in blood glucose levels of an animal induced by administering a test substance that is a candidate Cdk5 inhibitor to a non-human animal.

Fig. 1

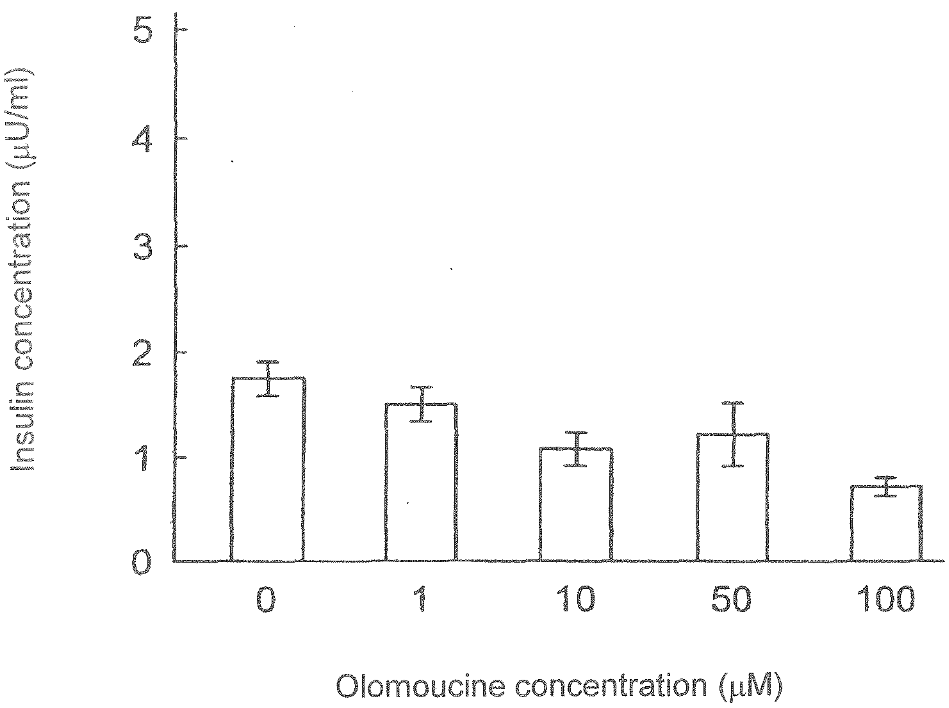
5
4
3
2
1
0
Insulin concentration (μU/ml)
0
1
10
50
100
Olomoucine concentration (μM)

Fig. 2

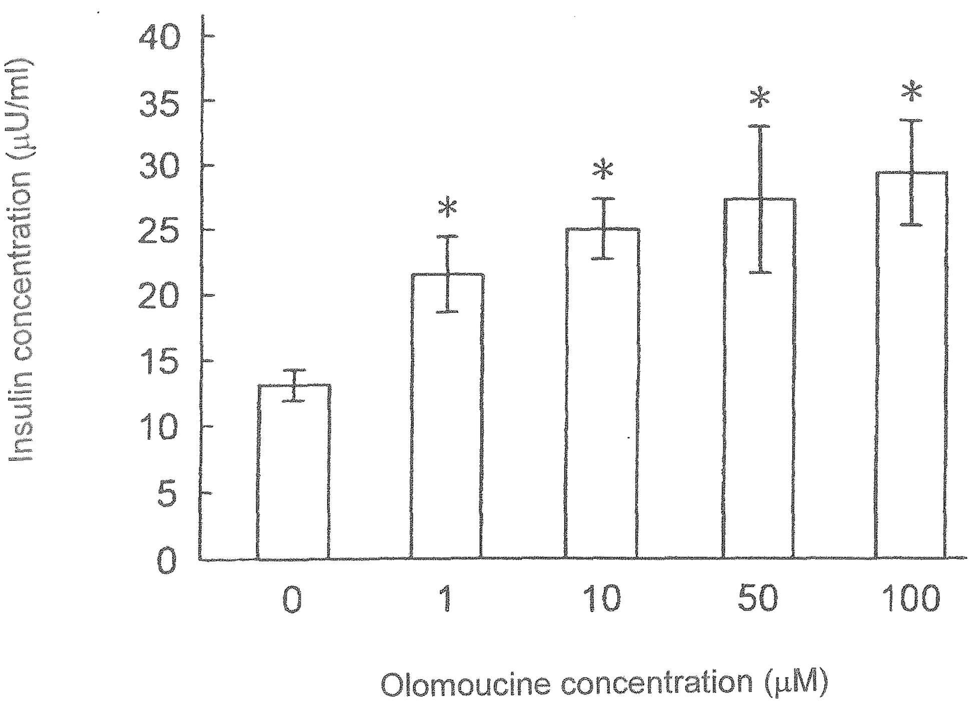
Insulin concentration (μU/ml)
40
35
30
25
20
15
10
5
0
*
*
*
*
0
1
10
50
100
Olomoucine concentration (μM)

Fig. 3

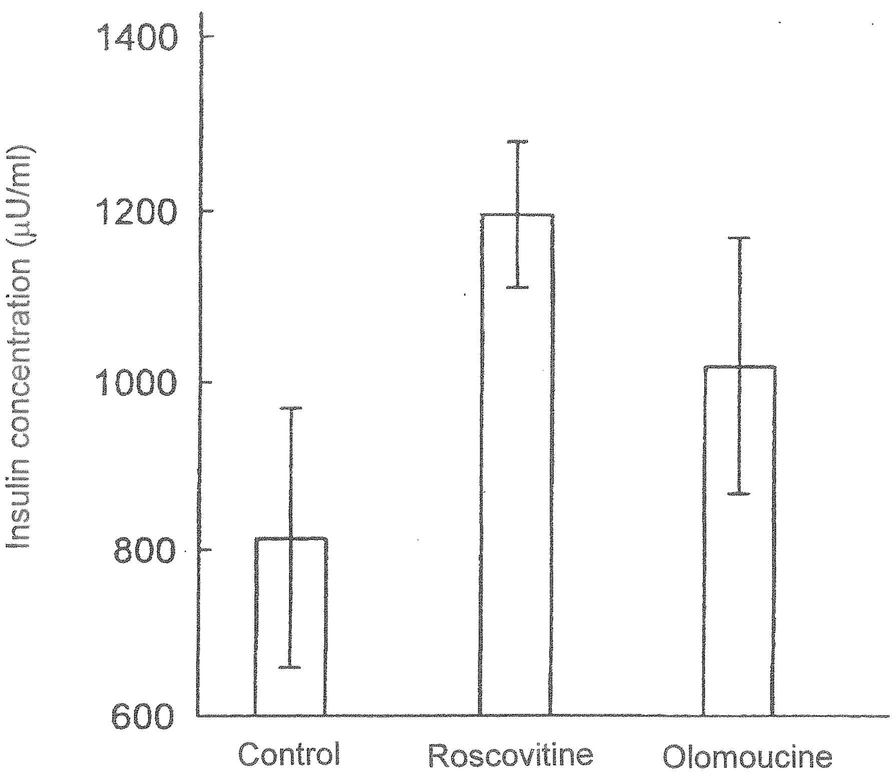
Insulin concentration (µU/ml)
1400
1200
1000
800
600
Control
Roscovitine
Olomoucine

Fig. 4

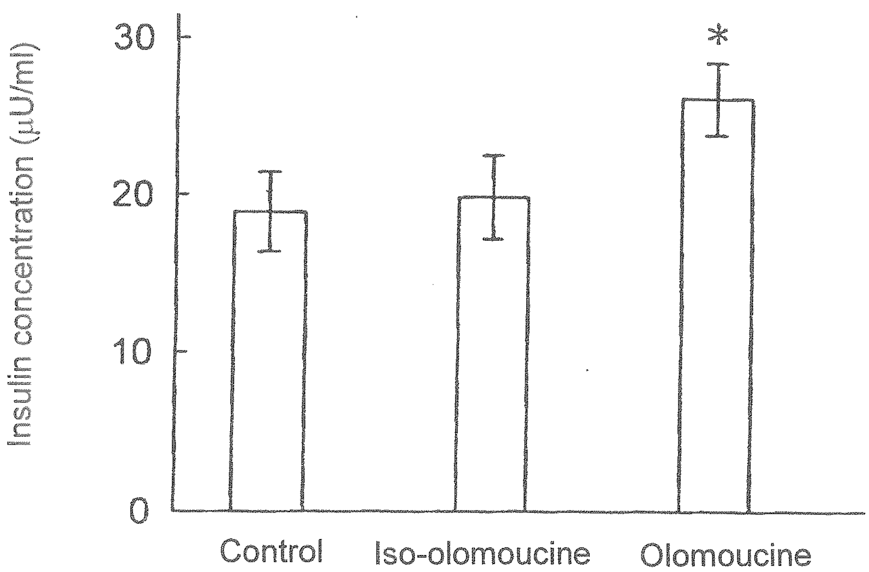
Insulin concentration (μU/ml)
30
20
10
0
*
Control
Iso-olomoucine
Olomoucine

Fig. 5

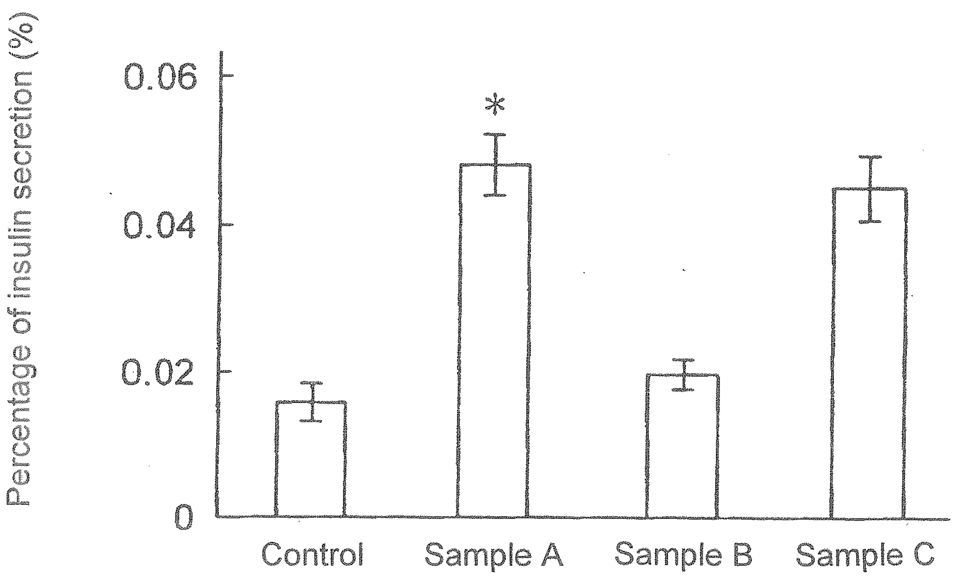
Percentage of insulin secretion (%)
0.06
0.04
0.02
0
*
Control
Sample A
Sample B
Sample C

Fig. 6a

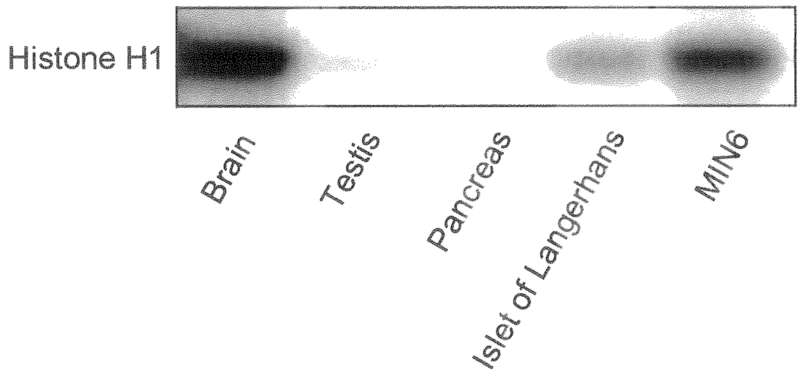

Fig. 6b

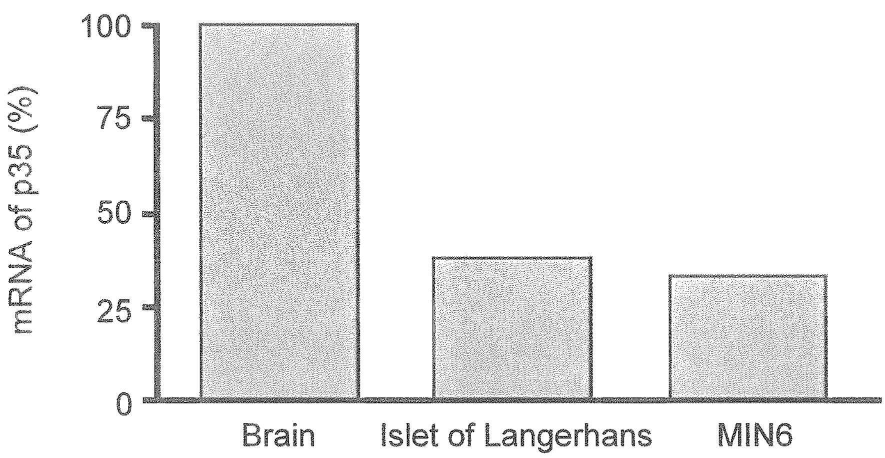

Fig. 6c

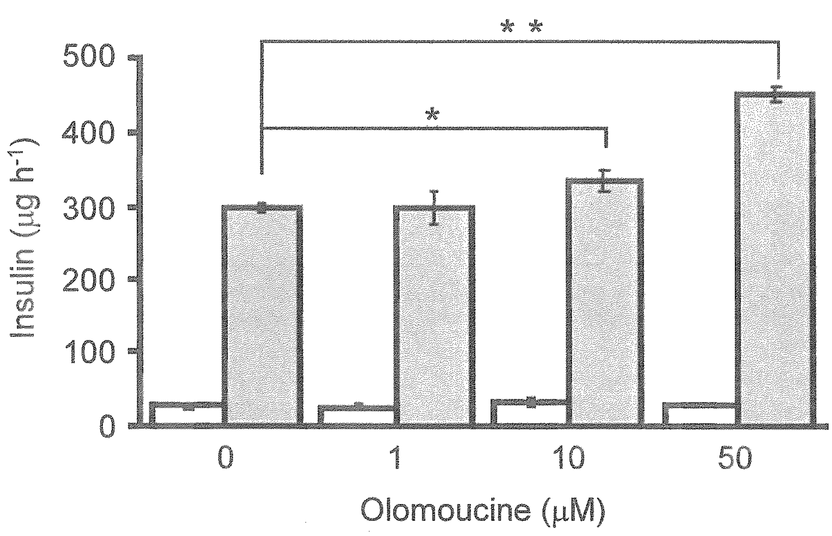

Fig. 6d

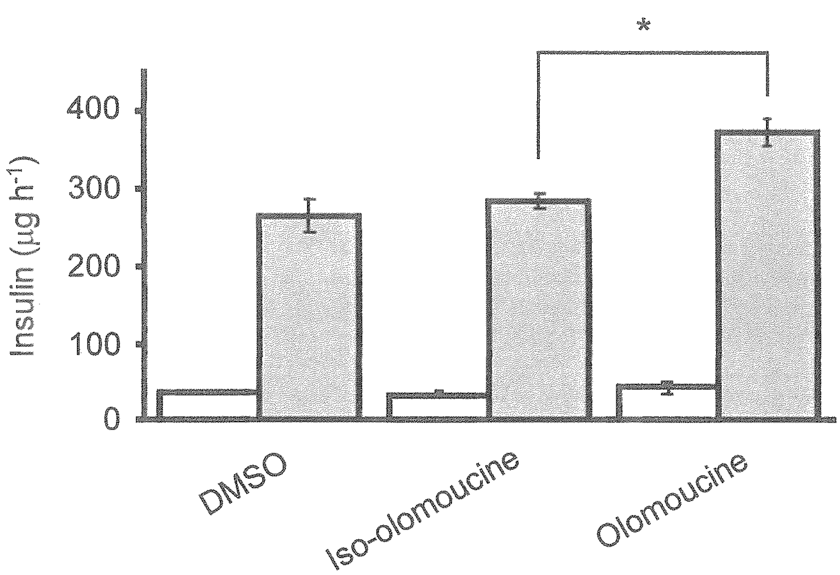
*
400
300
200
100
0
Insulin (μg h-1)
DMSO
Iso-olomoucine
Olomoucine

Fig. 6e

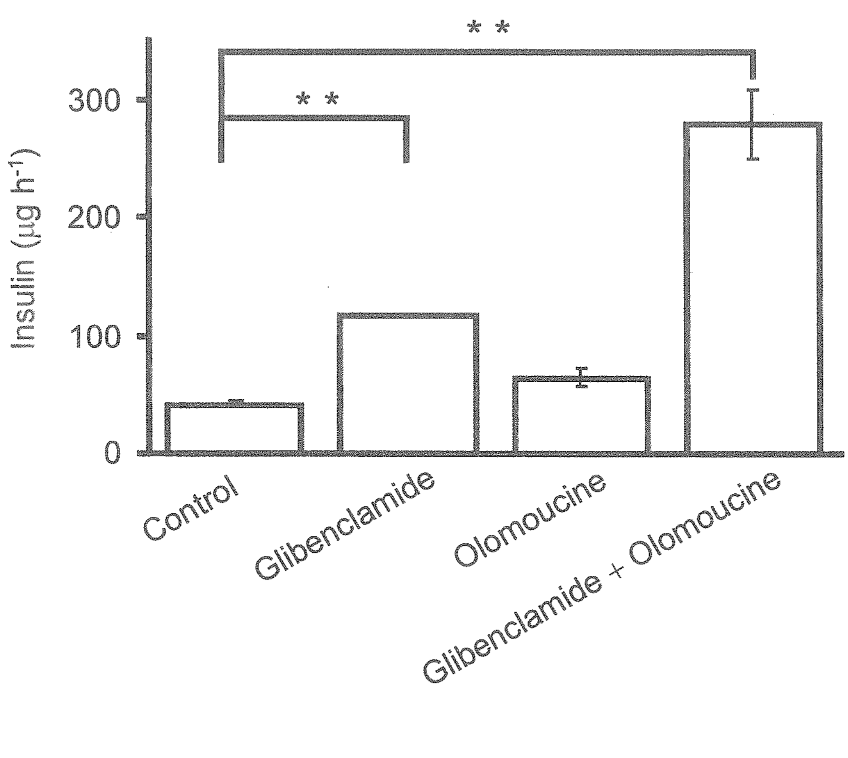
* *
* *
300
200
100
0
Insulin (μg h-1)
Control
Glibenclamide
Olomoucine
Glibenclamide + Olomoucine

Fig. 6f

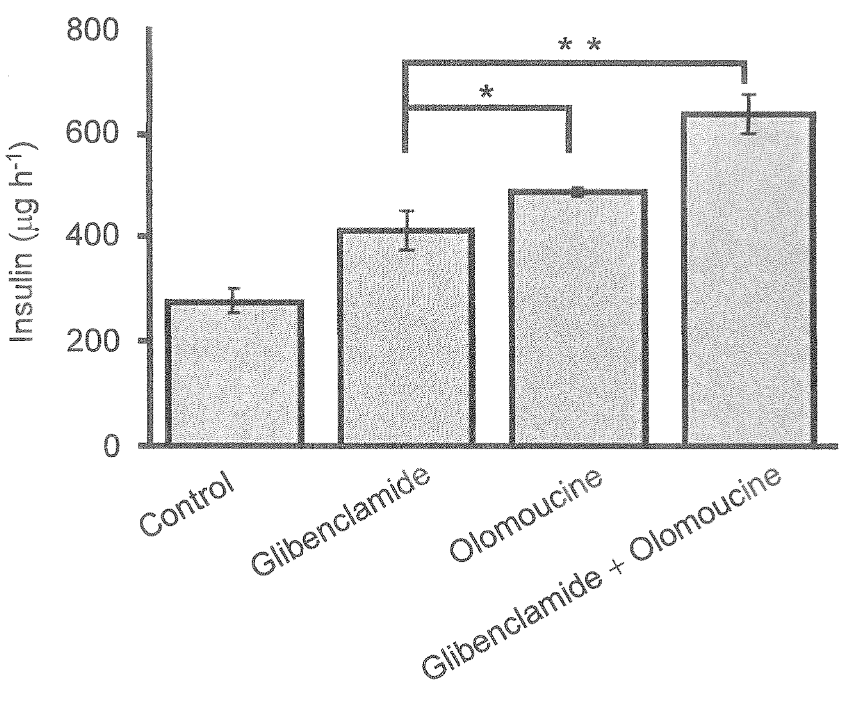
800
600
400
200
0
Insulin (μg h-1)
* *
*
Control
Glibenclamide
Olomoucine
Glibenclamide + Olomoucine

Fig. 6g

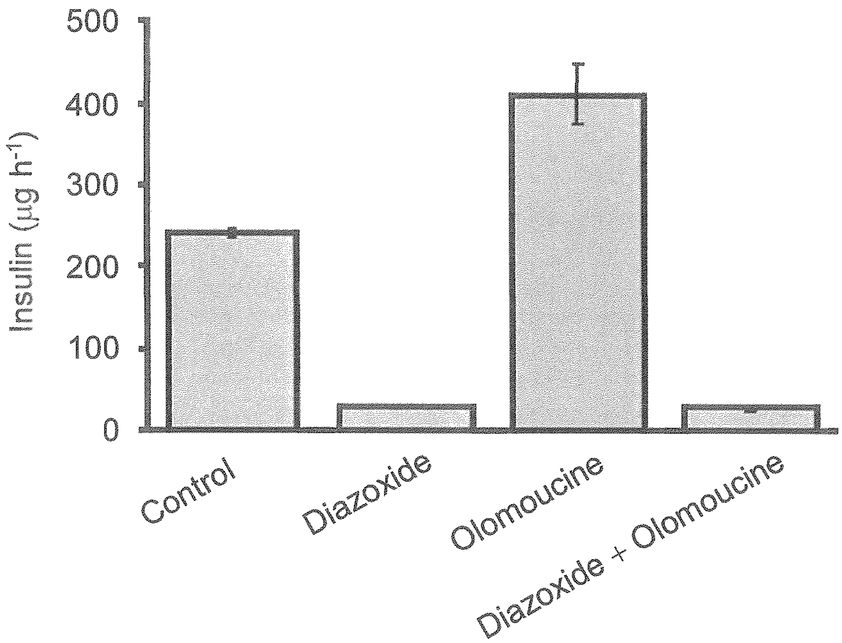
500
400
300
200
100
0
Insulin (μg h-1)
Control
Diazoxide
Olomoucine
Diazoxide + Olomoucine

Fig. 6h

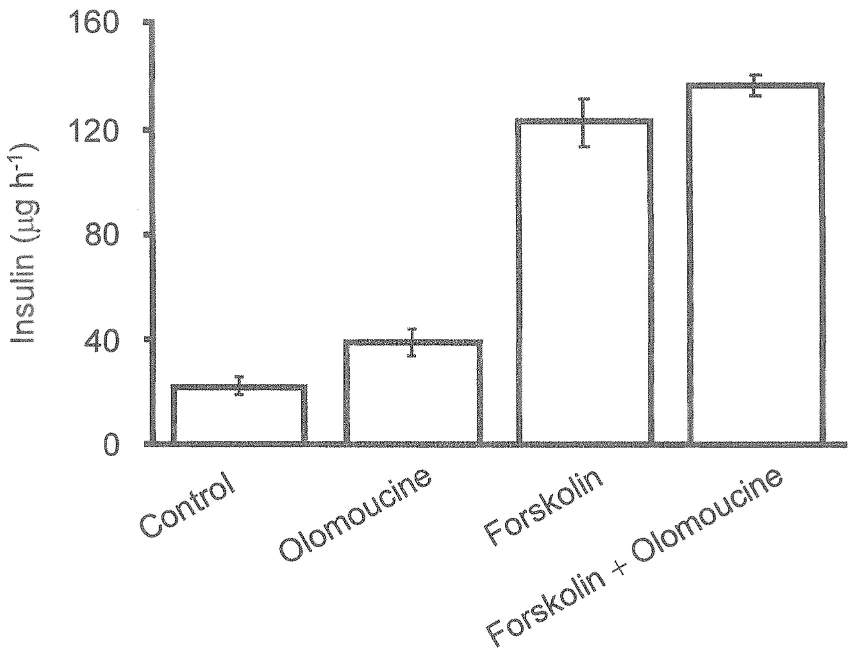
160
120
80
40
0
Insulin (μg h-1)
Control
Olomoucine
Forskolin
Forskolin + Olomoucine

Fig. 7a-1

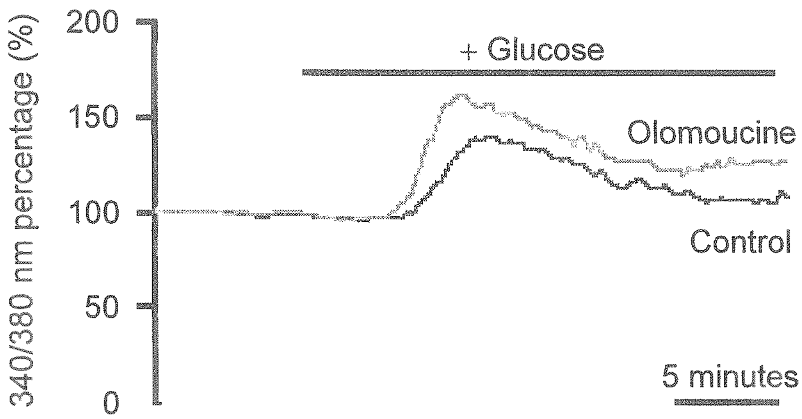
340/380 nm percentage (%)
200
150
100
50
0
+ Glucose
Olomoucine
Control
5 minutes

Fig. 7a-2

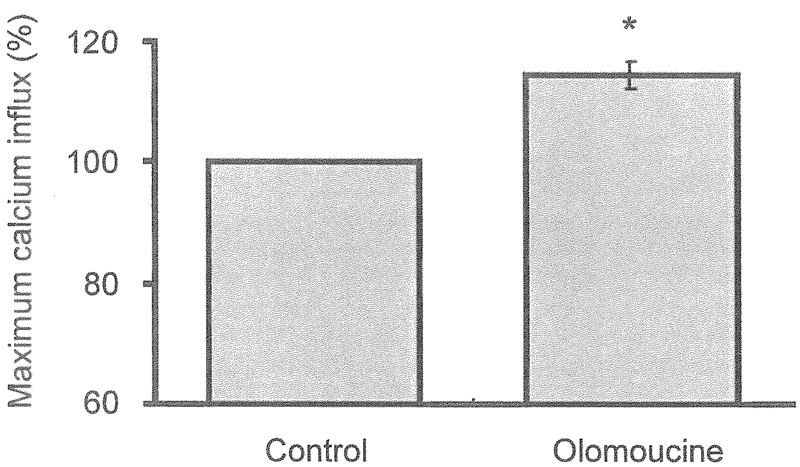

Fig. 7b-1

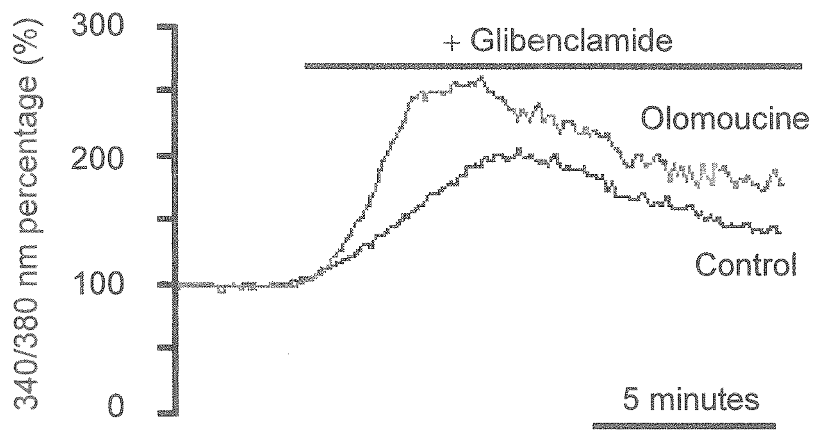
300
200
100
0
340/380 nm percentage (%)
+ Glibenclamide
Olomoucine
Control
5 minutes

Fig. 7b-2

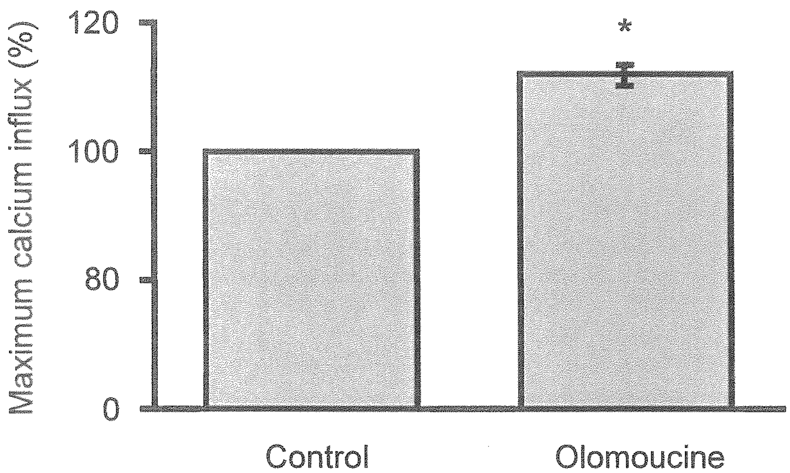

Fig. 7c

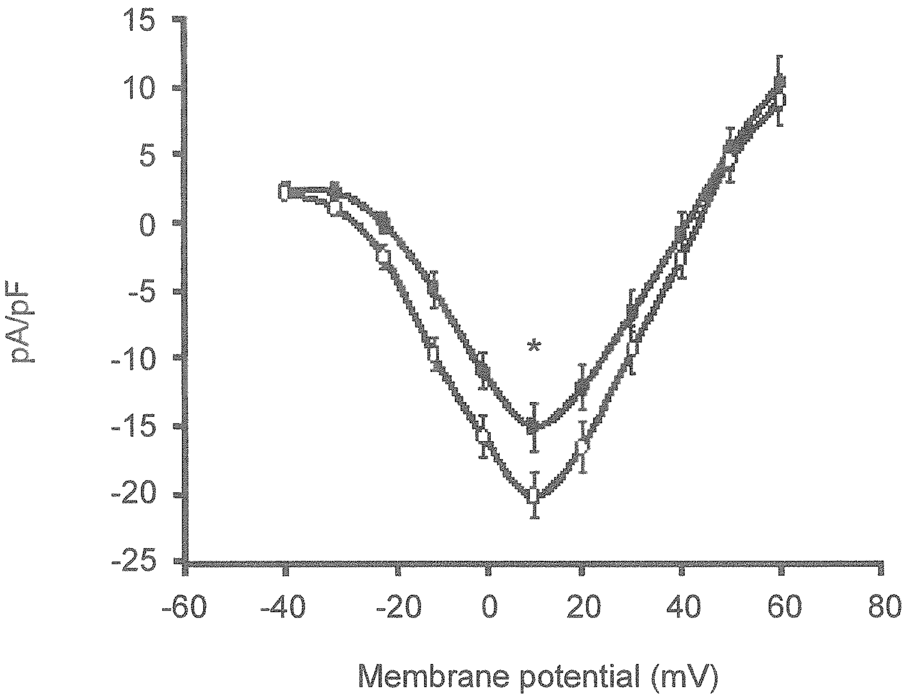
15
10
5
0
-5
-10
-15
-20
-25
pA/pF
*
-60
-40
-20
0
20
40
60
80
Membrane potential (mV)

Fig. 7d

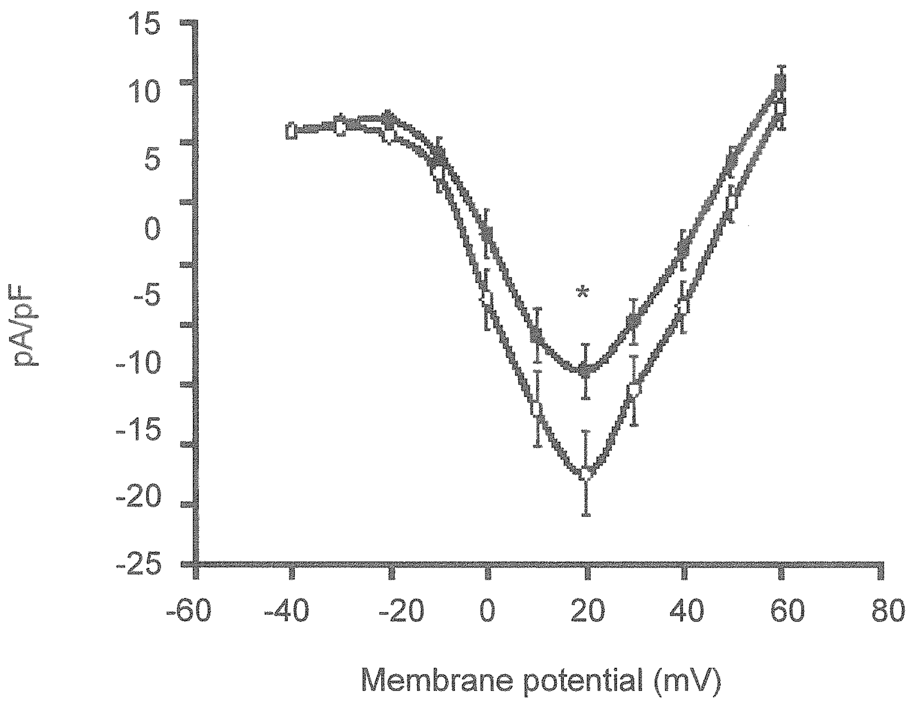
15
10
5
0
-5
-10
-15
-20
-25
pA/pF
*
-60
-40
-20
0
20
40
60
80
Membrane potential (mV)

Fig. 8a

H.spiens: 771 EEKERKKLAR TASPEKKQEL VEKPAVGESK

M.musculus: 771 EEKERKKLAR TASPEKKQEV MEKPAVEESK

R.norvegicus: 801 EEKERKKLAR TASPEKKQEV MEKPAVEESK

D.rerio: 782 EEKERKKLAR TASPEKRQNS EKPPLEDEKK

Fig. 8b

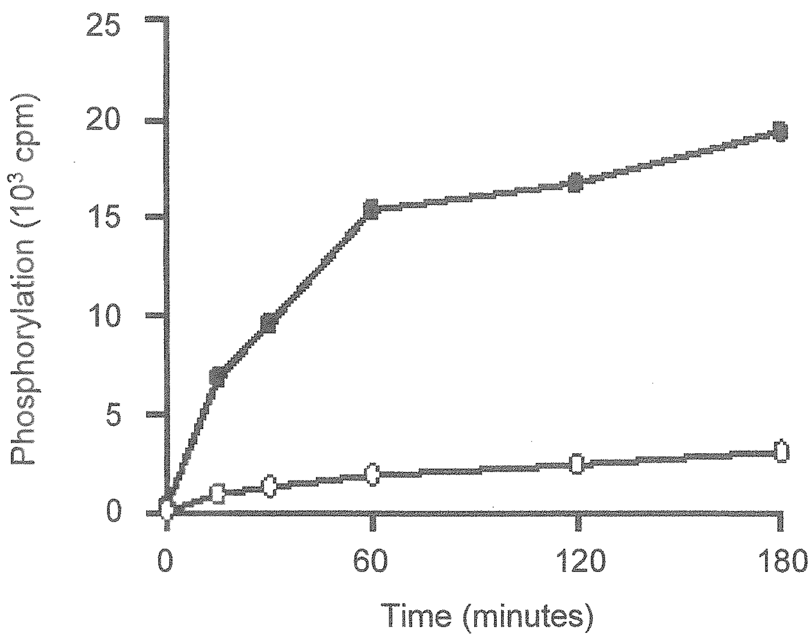
25
20
15
10
5
0
Phosphorylation ($10^3$ cpm)
0
60
120
180
Time (minutes)

Fig. 8c

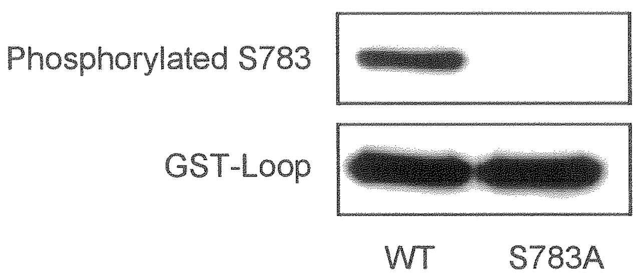

Fig. 8d

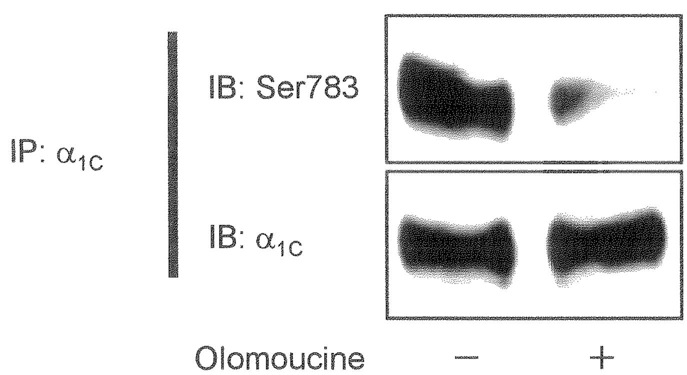

Fig. 8e

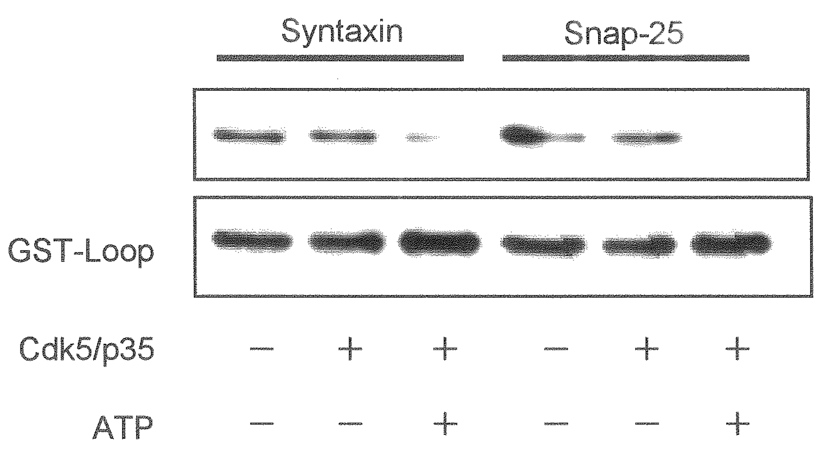

Fig. 8f

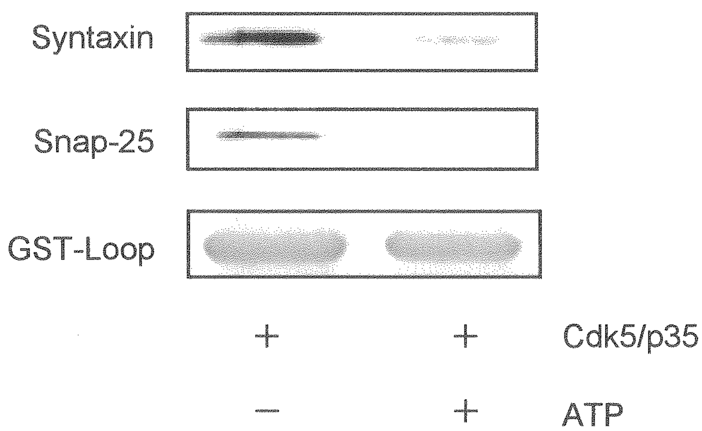

Fig. 9a

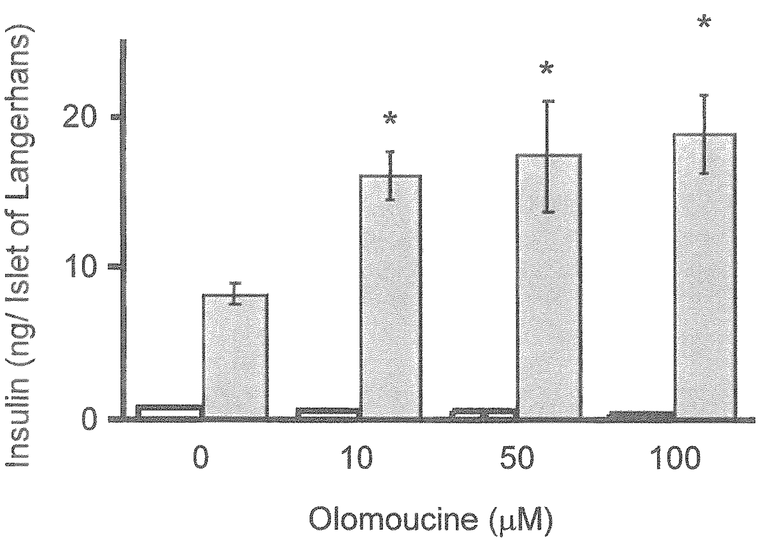
Insulin (ng/ Islet of Langerhans)
20
10
0
*
*
*
0
10
50
100
Olomoucine (μM)

Fig. 9b

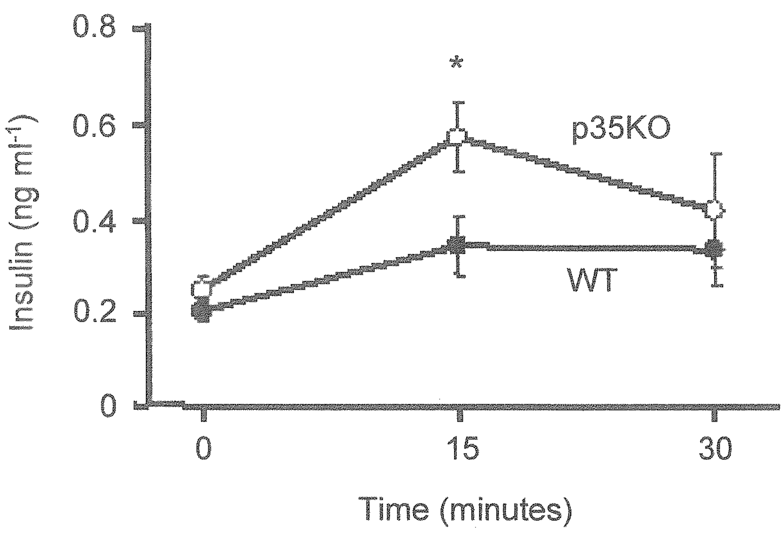

Fig. 9c

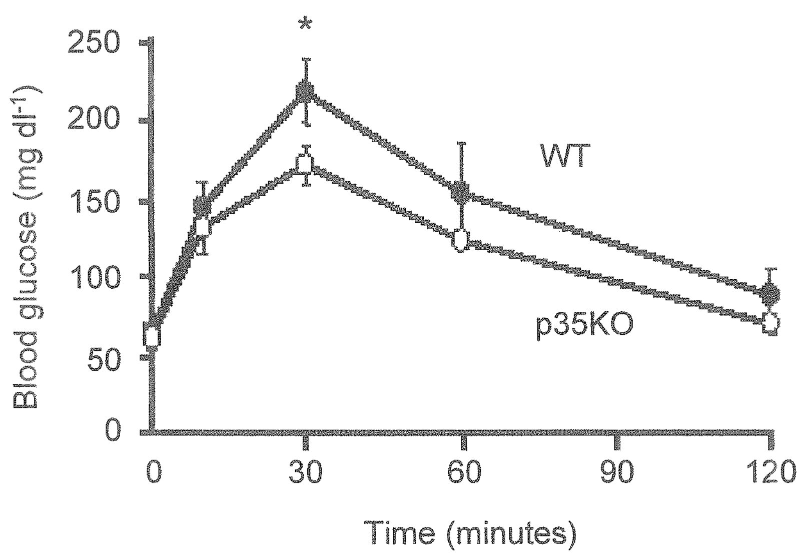
*
250
200
150
100
50
0
Blood glucose (mg dl-1)
WT
p35KO
0
30
60
90
120
Time (minutes)

Fig. 10

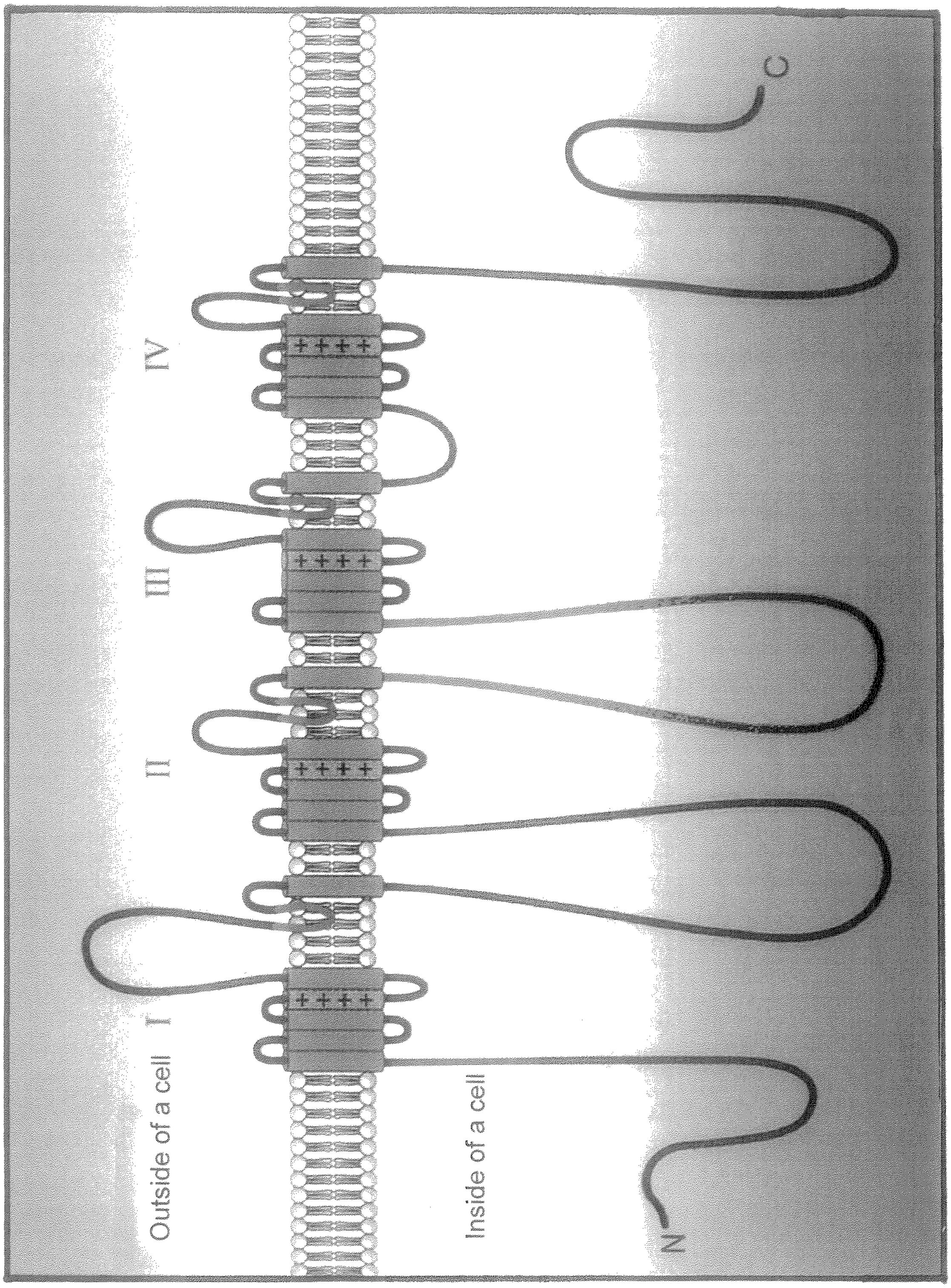

Fig. 11

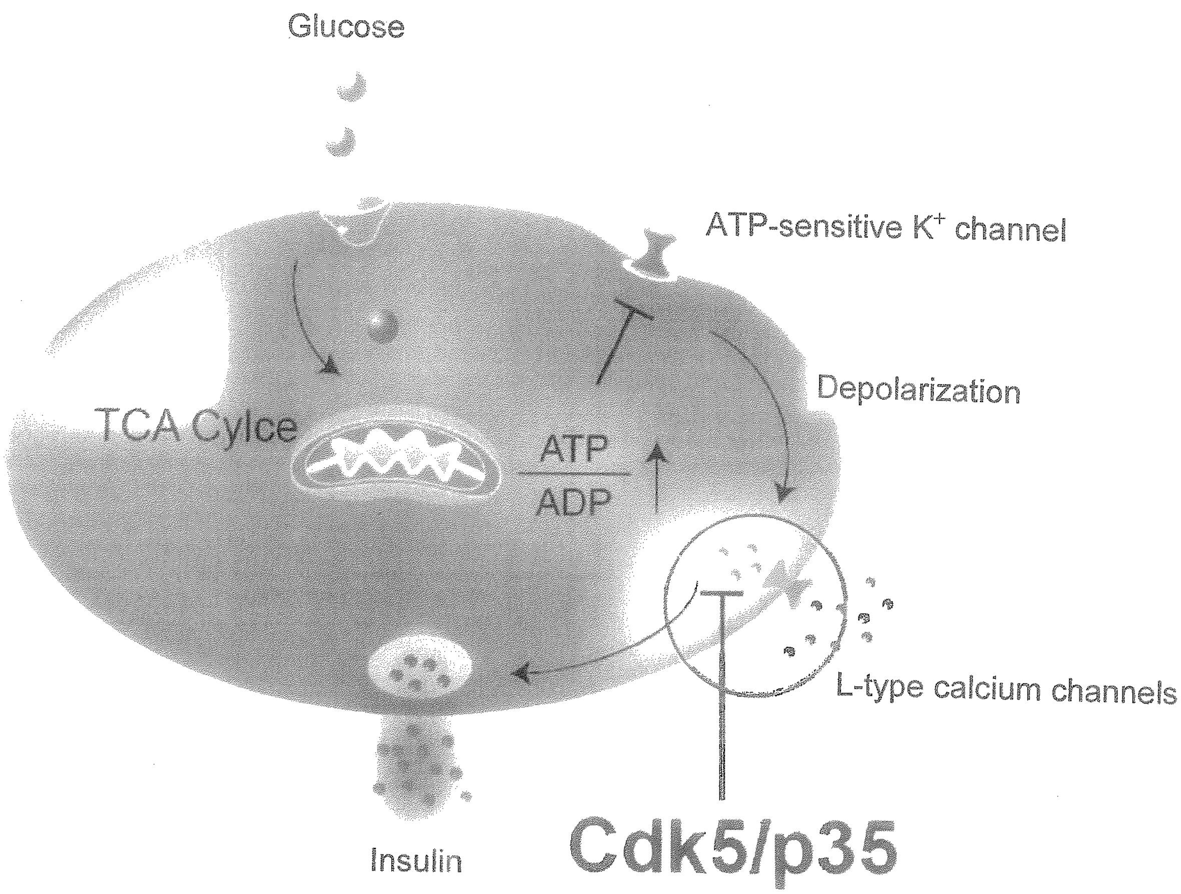
Glucose
ATP-sensitive K+ channel
Depolarization
TCA Cylce
ATP
ADP
L-type calcium channels
Insulin
Cdk5/p35

Fig. 12

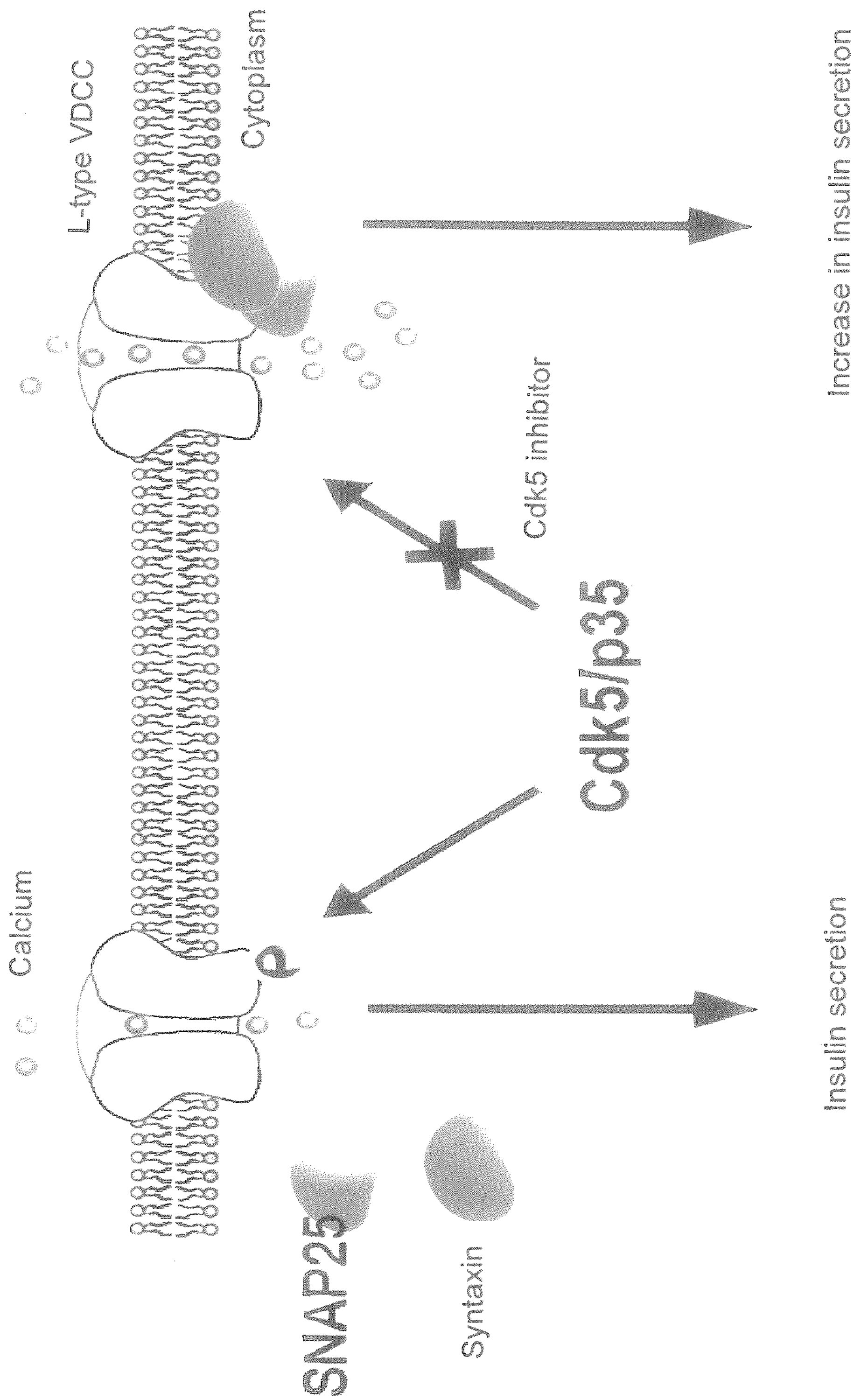
Calcium
L-type VDCC
Cytoplasm
Cdk5 inhibitor
Cdk5/p35
Increase in insulin secretion
Insulin secretion
SNAP25
Syntaxin

Fig. 13

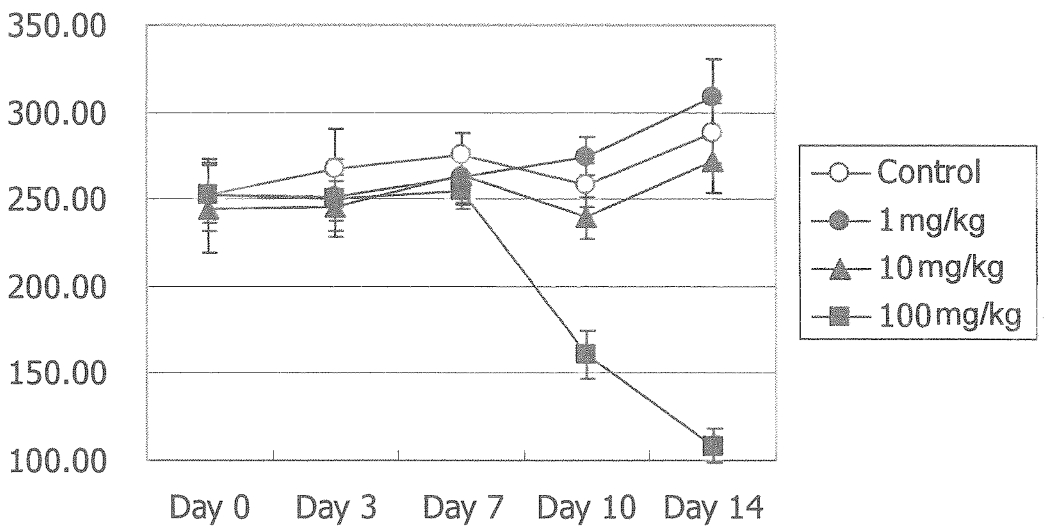
350.00
300.00
250.00
200.00
150.00
100.00
Day 0
Day 3
Day 7
Day 10
Day 14
Control
1 mg/kg
10 mg/kg
100 mg/kg

Fig. 14

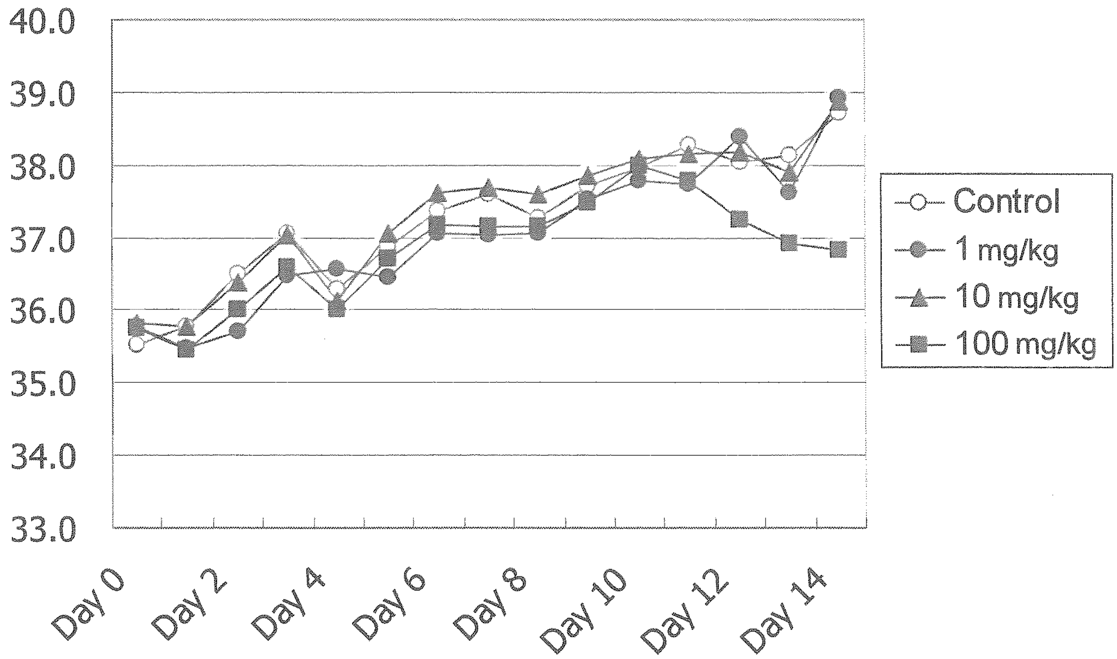
40.0
39.0
38.0
37.0
36.0
35.0
34.0
33.0
Day 0
Day 2
Day 4
Day 6
Day 8
Day 10
Day 12
Day 14
Control
1 mg/kg
10 mg/kg
100 mg/kg

**INTERNATIONAL SEARCH REPORT**

International application No.
PCT/JP2005/020870

A. CLASSIFICATION OF SUBJECT MATTER
***A61K45/00***(2006.01), ***A61K31/52***(2006.01), ***A61K38/00***(2006.01), ***A61P3/10***(2006.01), ***A61P43/00***(2006.01), ***C12Q1/02***(2006.01), ***G01N33/15***(2006.01), ***G01N33/50***(2006.01), *C07D473/16*(2006.01)

According to International Patent Classification (IPC) or to both national classification and IPC

B. FIELDS SEARCHED

Minimum documentation searched (classification system followed by classification symbols)
A61K31/00-45/08, A61P1/00-43/00, C12Q1/00-1/02, G01N33/00-33/50, C07D473/00-473/16

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), BIOTECHABS(STN), CAplus(STN), EMBASE(STN), MEDLINE(STN), SwissProt/PIR/Geneseq, JMEDPlus(JOIS), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| A | VESELÝ, J., et al., Inhibition of cyclin-dependent kinases by purine analogues.Eur.J. Biochem., 1994, 224(2), pages 771-786 | 1-16 |
| A | US 6693166 B1 (Hong Kong University of Science and Technology), 17 February, 2004 (17.02.04), (Family: none) | 1-16 |
| A | LILJA, L., et al., Cyclin-dependent Kinase 5 Promotes Insulin Exocytosis.J.Biol.Chem., 2001, 276(36), pages 34199-34205 | 1-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>17 January, 2006 (17.01.06) | Date of mailing of the international search report<br>07 February, 2006 (07.02.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No. PCT/JP2005/020870

C (Continuation). DOCUMENTS CONSIDERED TO BE RELEVANT

| Category* | Citation of document, with indication, where appropriate, of the relevant passages | Relevant to claim No. |
|---|---|---|
| A | UBEDA, M., et al., Glucose-Induced Exoression of the Cyclin-Dependent Protein Kinase 5 Activator p35 Involved in Alzheimer's Disease Regulates Insulin Gene Transcription in Pancreatic β-Cells. Endocrinol., 2004, 145(6), pages 3023-3031 | 1-16 |
| A | LILJA, L., et al., Cyclin-dependent Kinase 5 Associated with p39 Promotes Munc18-1 Phosphorylation and $Ca^{2+}$-dependent Exocytosis.J.Biol.Chem., 2004, 279(28), pages 29534-29541 | 1-16 |
| A | BRATANOVA-TOCHKOVA, T.K., et al., Triggering and Augmentation Mechanisms, Granule Pools, and Biphasic Insulin Secretion. Diabetes, 2002, 51(suppl.1), pages S83-S90 | 1-16 |
| A | YAN, Z., et al., Roscovitine: a novel regulator of P/Q-type calcium channels and transmitter release in central neurons. J. Physiol., 2002, 540(3), pages 761-770 | 1-16 |
| A | TABUCHI, A., et al., Differential Activation of Brain-derived Neurotrophic Factor Gene Promotors I and III by $Ca^{2+}$ Signals Evoked via L-type Voltage-dependent and N-Methyl-$_D$-asparate Receptor $Ca^{2+}$ Channels.J.Biol.Chem., 2000, 275(23), pages 17269-17275 | 1-16 |
| A | WO 03/082335 A1 (Sumitomo Pharmaceuticals Co., Ltd.), 09 October, 2003 (09.10.03), & AU 2003236354 A1 & US 2005/0119174 A1 | 14-16 |
| A | JP 2000-297015 A(Taisho Pharmaceutical Co., Ltd.), 24 October, 2000 (24.10.00), & WO 00/47172 A1 & AU 200024591 A | 14-16 |
| A | JP 2004-121241 A (Takeda Chemical Industries, Ltd.), 22 April, 2004 (22.04.04), & WO 2004/023870 A1 & AU 2003262020 A1 | 14-16 |
| P,X | WEI, F.-Y., et al., Cdk5-dependent regulation of glucosestimulated insulin secretion.Nat.Med., 2005, 11(10), pages 1104-1108 | 1-16 |
| P,X | JP 2004-339157 A (Kazuhito TOMIZAWA), 02 December, 2004 (02.12.04), (Family: none) | 1-16 |

Form PCT/ISA/210 (continuation of second sheet) (April 2005)

**INTERNATIONAL SEARCH REPORT**

International application No.
PCT/JP2005/020870

Claims 1 to 4 and 8 to 13

Drugs containing a Cdk5 inhibitor as the active ingredient are described in claims 1 to 4 and claims 11 to 13, while drugs containing a Cdk5 inhibitor as the active ingredient wherein the Cdk5 inhibitor is a peptidic substance are described in claims 8 to 10.

According to the term a Cdk5 inhibitor, an active ingredient is restricted based on its function and thus an extremely large number of chemicals having this property fall within the category thereof. However, even a person skilled in the art cannot definitely understand what specific chemicals correspond thereto. Even though the statement in the description is taken into consideration, no clear definition but some specific ingredients is merely presented. Accordingly, the statement makes the contents of the active ingredient unclear excluding those specified by specific chemical structures in claims 5 to 7, 9 and 10. That is, they are not supported by the statement of the description.

Although the active ingredient is restricted to peptidic substances in claims 8 to 13, specific effects thereof are not confirmed in the description excluding the low-molecular substances as set forth in claims 5 and 6. Since it is unclear whether or not peptidic substances having largely different chemical structures also show similar effects, the peptidic substances are not supported by the statement of the description.

Since the inventions according to these claims are not supported by the description (PCT Articles 5 and 6), the international search report was prepared by searching for prior art within the reasonable scope on the basis of the statement in the description.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004329165 A


### Non-patent literature cited in the description


- **TSAI, LH. et al.** *Development,* 1993, vol. 119, 1029-1040
- **LEW, J. et al.** *Nature,* 1994, vol. 371, 423-426
- **TSAI, LH. et al.** *Nature,* 1994, vol. 371, 419-423
- **TANG, D. et al.** *J. Biol. Chem.,* 1995, vol. 270, 26897-26903
- **CRUZ, JC et al.** *Curr. Opin. Neurobiol.,* 2004, vol. 14, 390-394
- **BIBB, JA. et al.** *Neurosignals,* 2003, vol. 12, 191-199
- **UBEDA, M. et al.** *Endocrinology,* 2004, vol. 145, 3023-3031
- **LILJA, L. et al.** *J. Biol. Chem.,* 2004, vol. 279, 29534-29541
- Stimulation of Insulin Release by RePaglinide and Glibenclamide Involves Both Common and Distinct Processes. *Diabetes,* 1998, vol. 47, 345-351
- New drug targets for type 2 diabetes and the metabolic syndrome. *Nature,* 2001, vol. 414, 821-827
- Inhibition of cyclin-dependent kinases by purine an alogues. *European journal of biochemistry,* 1994, vol. 224, 771-786
- Cyclin-dependent Kinase 5 Promotes Insulin Exocytosis. *The journal of biological chemistry,* 2001, vol. 276 (36), 34199-34205
- **INAGAKI, N. et al.** *Science,* 1995, vol. 270, 1166-1170
- **BRATANOVA-TOCHKOVA, TK. et al.** *Diabetes,* 2002, vol. 51, 83-90
- **GERBER, SH. et al.** *Diabetes,* 2002, vol. 51, 3-11
- **MOLLER, DE.** *Nature,* 2001, vol. 414, 821-827
- **YA-LI ZHENG et al.** *Eur. J. Biochem.,* 2002, vol. 26, 4427-4434
- **GOTO et al.** *Transplantaion,* 1987, vol. 43, 725-730
- **MAE et al.** *American Journal of Physiological Endocrinal Metabolism,* 2000, vol. 274, 773-781
- **LILLA, V. et al.** *Endocrinology,* 2003, vol. 144, 1368-1379
- **MINAMI, K. et al.** *Am. J. Physiol. Endocrinol. Metab.,* 2000, vol. 279, 773-781
- **WOLLHEIM, CB. et al.** *Methods Enzymol.,* vol. 192, 188-223
- **BEGUIN, P. et al.** *Nature,* 2001, vol. 411, 701-706
- **OHSHIMA, T. et al.** *Proc. Natl. Acad. Sci. U. S. A.,* 2001, vol. 98, 2764-2769
- **OHSHIMA, T. et al.** *Genomics,* 1996, vol. 35, 372-375
- **TOMIZAWA, K. et al.** *J. Cell. Biol.,* 2003, vol. 163, 813-824
- **VESELY, J. et al.** *Eur. J. Biochem,* 1994, vol. 224, 771-786
- **RENSTROM, E. et al.** *J. Physiol.,* 1997, vol. 502, 105-118
- **TOMIZAWA, K. et al.** *J. Neurosci.,* 2002, vol. 22, 2590-2597
- **DE, JONGH, KS. et al.** *Biochemistry,* 1996, vol. 35, 10392-10402
- **GAO, T. et al.** *Neuron,* 1997, vol. 19, 185-196
- **MCHUGH D et al.** *Proc. Natl. Acad. Sci. U. S. A.,* 2000, vol. 97, 12334-12338
- **SCHULLA, V. et al.** *EMBO. J.,* 2003, vol. 22, 3844-3854
- **YOKOYAMA, CT. et al.** *J. Neurosci.,* 1997, vol. 17, 6929-6938